# EUROPEAN PATENT APPLICATION

(11) **EP 3 932 402 A1**
(43) Date of publication of application: **05.01.2022**
(21) Application number: 20183093.2
(22) Date of filing: 30.06.2020
(51) Int. Cl.: A61K 31/341, A61K 31/451, A61K 31/496, A61K 31/54, A61K 39/00, A61K 45/06, A61P 31/14

(54) **ADAM17 INHIBITORS FOR USE IN THE PREVENTION AND/OR TREATMENT OF COVID-19**

(71) Applicant: Max-Delbrück-Centrum für Molekulare Medizin in der Helmholtz-Gemeinschaft, 13125 Berlin (DE)
(72) Inventor: de la Rosa, Kathrin, 10437 Berlin (DE)
(74) Representative: Kador & Partner PartG mbB

(57) **Abstract**

The invention relates to an ADAM17 inhibitor for use in the prevention and/or treatment of COVID-19 in a subject.

The invention further relates to a pharmaceutical composition comprising an ADAM17 inhibitor as an active ingredient for use in the prevention and/or treatment of COVID-19 in a subject.

The invention further relates to a kit of parts for use in the prevention and/or treatment of COVID-19 in a subject comprising i) an ADAM17 inhibitor and ii) a vaccine.

The invention further relates to a pharmaceutical composition comprising an ADAM17 inhibitor and an active immunization vaccine.

The invention further relates to a kit of parts comprising i) an ADAM17 inhibitor and ii) an active immunization vaccine.

The invention further relates to a method of preventing and/or treating COVID-19 in a subject, in the need thereof.

## Description

The invention relates to compounds for use in the prevention and/or treatment of COVID-19 in a subject. The invention further relates to pharmaceutical compositions for use in the prevention and/or treatment of diseases COVID-19 in a subject. The invention further relates to a kit of parts for use in the prevention and/or treatment of diseases COVID-19 in a subject.

The invention further relates to pharmaceutical compositions comprising an ADAM17 inhibitor and an active immunization vaccine. The invention further relates to a kit of parts comprising i) an ADAM17 inhibitor and ii) an active immunization vaccine.

The invention further relates to methods of preventing and/or treating COVID-19 in a subject.

The severe acute respiratory syndrome coronavirus (SARS-CoV) first emerged 17 years ago. In December 2019, a novel coronavirus (SARS-CoV-2) crossed species barriers to infect humans and was effectively transmitted from person to person, leading to a pneumonia outbreak first reported in Wuhan, China. This virus causes coronavirus disease-19 (COVID-19) with influenza-like symptoms ranging from mild disease to severe lung injury and multi-organ failure, eventually leading to death, especially in older patients with other co-morbidities. Severe acute respiratory coronavirus 2 (SARS-CoV-2) has become a worldwide pandemic but an effective therapy for COVID-19, the illness caused by the virus, is missing. Upcoming therapeutic strategies include compounds developed for the treatment of HIV, Influenza and Ebola infections. Other approaches are passive immunization strategies with plasma therapy and neutralizing antibodies, as well as the administration of recombinant Angiotensin-converting enzyme 2 (Ace2)-receptor, which has been shown to intercept the virus in *in vitro* cell culture. So far, no drug or vaccine has been approved for COVID-19 treatment or prevention and after the first pandemic wave, resurgence in contagion has been estimated for as late as 2024 (Kissler et al., 2020).

There is thus a need and accordingly it is the object of the present invention to provide new therapies for the prevention and/or treatment of COVID-19.

The present invention provides the new therapeutic approach to target the ADAM17 protease in order to prevent and/or alleviate COVID-19.

The present invention therefore further provides an ADAM17 inhibitor for use in the prevention and/or treatment of COVID-19 in a subject.

The present invention therefore provides a pharmaceutical composition comprising said an ADAM17 inhibitor as an active ingredient for use in the prevention and/or treatment of COVID-19 in a subject.

The invention further provides a kit of parts for use in the prevention and/or treatment of COVID-19 in a subject comprising i) an ADAM17 inhibitor and ii) a vaccine.

The invention further provides a pharmaceutical composition comprising an ADAM17 inhibitor and an active immunization vaccine.

The invention further provides a kit of parts comprising i) an ADAM17 inhibitor and ii) an active immunization vaccine.

The invention further provides a method of preventing and/or treating COVID-19 in a subject, the method comprising administering a therapeutically effective amount of an ADAM17 inhibitor to a subject in the need thereof.

Specifically, ADAM17 inhibitors can be used to prevent COVID-19 by inducing protective antibodies in SARS-CoV-2 non-infected subjects during vaccination. Furthermore, ADAM17 inhibitors can be used to prevent COVID-19 or at least prevent severe developments of COVID-19 by supporting the generation of protective antibodies in SARS-CoV-2 infected subjects. Still further, ADAM17 inhibitors can serve as a symptomatic treatment of COVID-19. Specifically, ADAM17 inhibitors can be used to ameliorate inflammatory and vasoconstrictive symptoms of COVID-19.

The ADAM17 inhibitors can be used for fostering the antibody responses to crucial SARS-CoV-2 targets. Specifically, it can foster the production of neutralizing antibodies against SARS-CoV-2 spike protein. Furthermore, ADAM 17 inhibitors can provide or increase protective immunity upon SARS-CoV-2 infection and thus can provide an option to close a critical gap before a vaccine becomes available. Still further, ADAM17 inhibitors combined with vaccine can improve the vaccine specific antibody response.

The ADAM metallopeptidase domain 17 (ADAM17), also called TACE (tumor necrosis factor-alpha-converting enzyme), is a 70-kDa enzyme that belongs to the ADAM protein family of disintegrins and metalloproteases. ADAM17 is an 824-amino acid polypeptide. ADAM17 is involved in the processing of a diverse variety of membrane-anchored cytokines, cell adhesion molecules, receptors, ligands, and enzymes, such as Angiotensin converting enzyme 2 (Ace2) and Tumor necrosis factor alpha (TNF-alpha). This process, which is also known as "shedding", involves the cleavage and release of a soluble ectodomain from membrane-bound pro-proteins, and is of known physiological importance. Thus ADAM17 is a "sheddase".

Angiotensin converting enzyme 2 (Ace2) has been identified as a key receptor for SARS-CoV-2 infections. The Ace2 enzyme is expressed as a transmembrane protein and can be cleaved by the ADAM17 protease inducing its shedding into the extracellular space. More particularly, the ectodomain from Ace2 is cleaved of and released into the extracellular space termed soluble Ace2 or sAce2. The 2002/2003 pandemic virus SARS-CoV-1 has been shown to modulate ADAM17 activity thereby increasing receptor shedding and downregulating surface Ace2 expression. The SARS-coronavirus spike proteins bind with very high affinity to soluble and membrane Ace2, the latter of which mediates virus uptake. The induction of antibodies that interfere with Ace2-spike interaction are crucial to develop a neutralizing response and confer protective immunity. However, activation of B cells and affinity maturation of potent antibodies strongly depends on the availability of the antigen, i.e. the spike proteins, because masking of protein structures was shown to abrogate efficient antibody responses. Given the high concentration of soluble Ace2 and its high affinity for the virus spike protein, shielding of the virus by its host receptor may render the most crucial virus epitopes unavailable to mount a proper antibody response.

In addition, local downregulation of Ace2 may promote an inflammatory environment because its reduced enzymatic activity leads to accumulation of its natural substrate Angiotensin II, which is proinflammatory and vasoconstrictive. TNF-alpha is a cell signaling protein (cytokine) involved in systemic inflammation and is one of the cytokines that make up the acute phase reaction. ProTNF-alpha is also a substrate of ADAM17 and TNF-alpha has been suggested to contribute to severe COVID-19. Decreased TNF-alpha release by protease inhibition may thus further ameliorate inflammation after SARS-CoV-2-infection.

Furthermore, subjects who are at high risk of developing severe COVID-19 symptoms often share the phenotypic characteristic of high abundance of the viral host receptor Ace2 in its soluble form.

It has been found that an ADAM17 inhibitor for use according to the invention reduces Ace2 shedding. Thereby, less soluble Ace2 is present in the extracellular space. Thus, epitope masking is reduced. Hence, the effect of using an ADAM17 inhibitor is to foster maturation of SARS-CoV-2 neutralizing antibodies in SARS-CoV-2 infected subjects, specifically the maturation of SARS-CoV-2 receptor-binding domain (RBD)-directed neutralizing antibodies. Moreover, the ADAM17 inhibitor for use provides a decrease of vasoconstrictive effects and the promotion of an anti-inflammatory environment by decreasing the level of vasoconstrictive Angiotensin II (Angll) and preventing pro-inflammatory TNF-alpha shedding. Subjects who are at high risk of developing severe COVID-19 symptoms may specifically benefit from the invention.

Accordingly, the ADAM17 inhibitor for use according to the invention provides new therapies for the prevention and/or treatment of COVID-19 in a subject.

### Detailed description

Neutralizing antibodies are crucial to elicit protective immunity after natural pathogen exposure or vaccination and are considered key for passive immunization strategies. Several studies support that protective antibody responses to lately emerging severe acute respiratory coronavirus 2 (SARS-CoV-2) can be weak. For instance, titers of virus particle neutralizing antibodies to SARS-CoV-2 were low (30%) or below detection limit (5.7%) in 175 individuals recovered from mild COVID-19 disease (Wu et al., 2020). In a study with 68 COVID-19 convalescent individuals, neutralizing antibody titers were undetectable in 18% of donors 30 days post symptom onset (Robbiani et al., 2020). In a different cohort of 173 COVID-19 hospitalized cases, the rise of antibody titers was not always accompanied by virus RNA clearance, in particular for 3 critical patients, despite 93.1% of patients being seropositive for antibodies binding to the virus spike protein (82.7% IgM+, 64.7% IgG+) (Zhao et al., 2020). A third cohort study of 222 hospitalized patients performing combined measurement of virus nucleocapsid and spike protein specific antibodies reported more frequent severe cases in patients with high (51.8%) compared to low (32.3%) IgG levels (p=0.008). (Zhang et al. 2020). Of note, antibodies raised against the nucleocapsid protein would not confer protection by virus neutralization.

The observation that antibody titers specific for SARS-CoV-2 do not necessarily correlate with protection from COVID-19 disease suggests that antibodies may not be raised against key virus targets. Cellular entry of SARS-CoV-2 depends on binding of the viral Spike by its receptor-binding domain (RBD) to the target receptor angiotensin converting enzyme 2 (Ace2). One mechanism by which antibodies confer neutralizing activity is by blocking virus uptake by binding to the RBD of the spike protein. In a recent study, 8 patients hospitalized with COVID-19 showed varying antibody titers against full length spike protein, and antibodies targeting the crucial receptor binding domain (RBD) were low in half of the patients. (Ju et al, 2020). Although isolated monoclonal antibodies from seropositive individuals showed very high binding affinities for RBD, they conferred only limited ability to compete with Ace2 receptor binding. Another recent study reported that 19/26 COVID-19 sera showed intermediate to high reactivity to full length spike, 10/26 and 1/26 showed intermediate and high binding to the RBD, respectively, and only 3/26 sera were inhibiting Ace2 binding to RBD (Chen et al., 2020).

A surprising finding was reported by a comprehensive study involving 1850 COVID-19 patients from Wuhan, China. Survivors showed a gradual increase of Nucleocapsid, full spike and RBD specific IgG antibodies. By contrast, non-survivors displayed highly elevated IgG antibody titers to full spike and nucleocapsid in the first 3 weeks compared to survivors but, importantly, non-survivors showed very poor levels of RBD specific IgG up to 3-4 weeks after symptom onset (Li et al. 2020).

Limited access to an antigen by masking of an epitope can downregulate B cell responses and antibody affinity maturation (Zhang et al., 2013; Bergstrom et al., 2017; Zarnitsyna et al., 2015; Meyer-Hermann, 2019). The coronavirus receptor Ace2, which is expressed as a transmembrane protein, can be cleaved by proteases inducing its shedding into the extracellular space. Ace2 shedding was reported to occur in human airway epithelia, which is a site of SARS-CoV-1 (Jia et al., 2009) and SARS-CoV-2 infection. Moreover, SARS-CoV-1 was shown to increase Ace2 shedding via modulation of the TNF-alpha converting enzyme (TACE or ADAM17) thereby downregulating Ace2 surface expression (Haga et al., 2008). A putative ADAM17 recognition motif was identified by a L584A Ace2-mutant which attenuated shedding and facilitated SARS-CoV entry into target cells (Jia et al., 2009). ADAM17 was found to compete with TMPRSS2, a protease that requires Ace2 amino acids 697 to 716 for cleavage. TMPRSS2 not only augments SARS-CoV-1 and -2 entry into host cells by targeting Ace2 but also by cleaving and activating the virus spike protein for membrane fusion (Heurich et al., 2014; Hoffmann et al., 2020).

It is known that masking of epitopes can suppress the generation of specific antibodies. For example, passively administered IgG with specificity for either the immunogen or for the idiotypic determinant can interfere with the induction of an antibody response. Fc-receptors were shown to play important roles in antibody feedback and immune response modulation (Nimmerjahn and Ravetch, 2010), however, it was suggested that the main mechanisms by which antibodies suppress responses is masking of epitopes preventing antigen recognition by specific B cells. How small amounts of administered antibodies can abolish primary IgM responses (Henry and Jerne, 1968; Heyman and Wigzell, 1984; Karlsson et al., 1999; 2001a; Bruggemann and Rajewsky, 1982; Moller, 1985) and IgG responses (Heyman and Wigzell, 1985; Brinc et al., 2007; Getahun and Heyman, 2009; Nicholas and Sinclair, 1969; Safford and Tokuda, 1971) has been intensively studied over the years. Importantly, the suppression effect of antibodies can be observed in mice lacking the Fcγ-receptors FcγRI, FcγRIII, FcyRIV (FcRy KO), the inhibitory FcγRIIB, and also the neonatal Fc receptor (FcRn) (β2-microglobulin KO) (Karlsson et al., 1999; 2001a; Bernardo et al., 2015; Bergstrom and Heyman, 2015). Furthermore, the regulatory effects of antibodies was found not to function via the constant part or a particular IgG subclass (Reth et al., 1981) nor IgE (Karlsson et al., 1999; 2001b). In line with this observations, administered F(ab')2 fragments were able to suppress (Tao and Uhr, 1966; Cerottini et al., 1969; Bernardo et al., 2015; Karlsson et al., 1999).

Likewise, soluble Ace2 may mask SARS-CoV-2 spike protein thereby shielding crucial epitopes from induction of neutralizing antibodies. In certain tissues, local concentration of soluble Ace2 may be even higher, especially if shedding is increased after SARS-CoV-2 virus infection. In conclusion, epitope masking by soluble Ace2 is one explanation why antibodies to SARS-CoV-2 lack neutralizing capabilities in certain individuals.

Mathematical models have suggested that epitope masking and enhanced antibody mediated clearance can synergize to suppress humoral immune responses (Daëron and Lesourne, 2006). Other in silico strategies have been used to tune an immune response towards low accessible antigens by modelling an application of high affinity antibodies (>5 nMol) early during the immune response (Meyer-Hermann, 2019). *In vivo* B cell selection was shown to be restricted by administration of high affinity IgM antibodies (90 µg/mouse) (Zhang et al., 2013). In a sheep red-blood cell mouse model as few as 0.4 ug injected IgG/mouse significantly suppressed antibody responses while 1 µg/mouse was needed when Fab fragments were administered. (Karlsson et al., 1999). While IgM and IgD were shown to weakly suppress antibody responses at doses as high as 100 µg/mouse, high affinity IgGs (10⁻⁸ and 10⁻⁹ M) could inhibit the response already at 2 µg/mouse, a finding that was attributed to a shorter half-life of IgM and IgD antibodies (Brüggemann and Rajewsky, 1982). The same study also described that the response to multideterminant immunogens can be suppressed by binding of a single antibody species to only one epitope. This finding has been interpreted as an evidence for Fc-receptor dependence. However, a lack of epitope specific suppression could also be explained by steric hindrance (Bergström et al., 2017; Karlsson et al., 1999; Heyman, 1999). If a high density epitope is bound by antibodies this may also mask non-specific neighboring epitopes.

In summary, numerous studies have shown that low amounts of administered antibodies specific for a certain antigen are efficiently masking epitopes during immunization and suppress the generation of specific antibodies to this antigen. Similarly, already low amounts of Ace2 may mask SARS-CoV-2 spike proteins.

Binding affinities for SARS-CoV-2 to Ace2 were reported to be 4- to 20-fold increased when compared to SARS-CoV-1. The trimeric pre-fusion SARS-CoV-2 spike ectodomain (residues 1-1208) binds the Ace2 receptor with an equilibrium dissociation constant (Kd) of ∼15 nM, while SARS-CoV-1 spike has a Kd of 325 nM (Wrapp et al., 2020). The same study determined SARS-CoV-2 spike RBD binding (residues 319-591) at a Kd of 34.5 nM. Other reports detected higher binding affinities of the RBD to Ace2 with 4.7 nM versus 31 nM (Lan et al., 2020) and 1.2 nM versus 5 nM, for SARS CoV-2 and SARS CoV-1, respectively (Walls et al., 2020). Using transiently transfected HEK cells expressing human Ace2, binding of Fc-fused spike RBDs was determined at a 50% effective dose (EC50) of 0.14 µg/ml (SARS-CoV-2) and 1.32 µg/ml (SARS-CoV-1) (Tai et al., 2020). The higher binding affinity of the virus protein to its target receptor may lead to an even enhanced epitope masking effect for SARS-CoV-2 compared to SARS-CoV-1.

If spike proteins would be masked and shielded from B cell recognition, spike-protein specific antibodies may be of low abundance in SARS-CoV infected individuals. Sera collected during the SARS-CoV-1 outbreak of 2002/2003 have been extensively analyzed by applying serology assays detecting antibodies specific for full length or partial recombinant proteins. Nucleocapsid-protein specific Enzyme Linked Immunosorbent Assay (ELISA) assays applied to 7 different cohorts detected 80%, 89.1%, 89.6%, 92%, 94% and 2-times 100% positive samples, and Westernblot assays showed higher sensitivity in 6 different cohorts with 94.8% and 5x 100% of positive sera (reviewed in: (Meyer et al., 2014). By contrast, spike-protein specific ELISAs applied in 2 cohorts showed 13% and 60.2% positive sera. In 7 cohorts, 30%, 50%, 70%, 75%, 85%, 86.7%, 100% of individuals displayed spike specific antibodies detected by Westernblot; and only highly sensitive fluorescence assays could detect spike specific antibodies in all individuals of 2 cohorts. These results suggest that fewer individuals raise antibodies against the virus spike protein and/or at lower abundance compared to the intraviral nucleocapsid. However, a direct comparison is hampered as data were collected in independent studies using distinct methodological setups. Furthermore, nucleocapsid is the most abundant viral protein and also less divergent from other coronaviruses, increasing the odds of crossreactivity. A recent report analyzing 214 patients hospitalized with COVID-19 by ELISA detected nucleocapsid specific IgG in 70.1% of individuals, and even more patients (74.3%) were positive for IgG specific for the RBD of the spike protein. (Liu et al., 2020). The report did not provide a quantification of antibody titers and it remains elusive if spike specific IgG detected would be suitable to neutralize the virus.

If Ace2 shedding would interfere with B cell maturation, similar viruses that use Ace2 as a host-receptor but do not activate receptor shedding may induce a more potent spike specific antibody response. Interestingly, HNL63-CoV, which is a SARS-CoV related virus inducing the common cold, uses the Ace2 receptor for cell entry but does not mediate its shedding (Hofmann et al., 2005; Haga et al., 2008; Glowacka et al., 2010; Wu et al., 2009). Potent neutralizing antibodies directed to the HNL63-CoV spike protein were detected in all patient serum samples of 8 years and older (Hofmann et al., 2005). HNL63-CoV and SARS-CoV-1 have no structural homologies in the spike RBD, but both viruses detect a common hotspot on Ace2 receptor with similar affinity (Wu et al., 2009).

It should be noted, that other mechanisms can limit recognition of the coronavirus spike protein by the immune system such as conformational masking and glycan shielding. SARS-CoV-1 and -2 as well as MERS-CoV spike proteins exist in distinct conformations (Song et al., 2018; Walls et al., 2017; 2019; 2020). Closed spike trimer configurations were found for HNL63-CoV (Walls et al., 2016), rendering RBD a hidden determinant before the opening of HNL63-CoV spike trimers (Walls et al., 2020). Nevertheless, such structural masking does not interfere with the generation of a potent neutralizing response against the HNL63-CoV spike protein.

Hence, in the case of SARS-CoV-2, the antibody responses may be poorly neutralizing due to Ace2 receptor shedding, because high affinity binding of soluble Ace2 to the spike protein masks virus recognition by the immune system and interferes with B cell activation and affinity maturation.

Therefore, an ADAM17 inhibitor for use according to the invention by reducing Ace2 shedding may foster maturation of neutralizing antibodies by minimizing epitope masking. Accordingly, the ADAM17 inhibitor can be used for improved vaccination and thus in the prevention of an infection with SARS-CoV-2 and thus for preventing COVID-19 for SARS-CoV-2 non-infected subjects. Furthermore, the ADAM17 inhibitor can be used for improving the immune response in natural infections, i.e. is used for preventing any COVID-19 symptoms, for preventing mild COVID-19 symptoms or for preventing severe COVID-19 symptoms in SARS-CoV-2 infected subjects.

Classical vaccination approaches induce immunity by the application of recombinant proteins or inactivated pathogens, but there are potential drawbacks for SARS-CoV-2 vaccines including limited global production capacities. (reviewed in: (Amanat and Krammer, 2020). Other vaccination approaches include DNA and RNA vaccines. Despite the advantage of a rapid manufacturing process, there are concerns regarding safety and the need for specific delivery devices to reach immunogenicity. Since ADAM17 inhibitors for use may foster protective immunity during infection, this may close a critical gap in the ongoing pandemic before a vaccine becomes available for large immunization campaigns. Moreover, it can be used to support a vaccine induced antibody response.

Hence, the ADAM17 inhibitor for use according to the invention prevents and/or treats COVID-19 in a subject. Preferably, the subject is human.

Accordingly, the subject may be a SARS-CoV-2 non-infected subject, preferably a human person or a SARS-CoV-2 infected subject, preferably a human person. A non-infected subject benefits from the ADAM17 inhibitor for use during vaccination, since the inhibitor supports a vaccine induced antibody response. An infected subject benefits from the ADAM17 inhibitor for use, since the inhibitor supports the SARS-CoV-2 induced antibody response.

A SARS-CoV-2 infection may be determined by any method known to the skilled person, for example by detecting the virus in a sample by a polymerase chain reaction based method, such as RT-PCR. The sample may be any one used in the art, for example blood, sputum, feces, urine, and nasal samples preferably nasopharyngeal swabs, nasal swabs, throat swabs, fibrobronchoscope brush biopsy or bronchoalveolar lavage fluid.

Preferably, the subject shows at least low abundance, more preferably very low abundance or even no SARS-CoV-2 neutralizing antibodies upon vaccination or natural infection. More preferably, the subject shows at least low abundance of SARS-CoV-2 spike protein specific neutralizing antibodies, more preferably SARS-CoV-2 RBD protein specific neutralizing antibodies, more preferably Ace2 binding site of the spike specific neutralizing antibodies. More preferably, the SARS-CoV-2 neutralizing antibodies, the SARS-CoV-2 spike protein specific neutralizing antibodies, the SARS-CoV-2 RBD protein specific neutralizing antibodies, or the Ace2 binding site of the spike specific neutralizing antibodies are IgG and/or IgA and/or IgM. Most preferably, the SARS-CoV-2 neutralizing antibody is a SARS-CoV-2 Ace2 binding site of the spike protein specific IgG and/or IgA and/or IgM.

Since SARS-CoV-2 spike protein specific and/or RBD specific and/or Ace2 binding site of the spike specific neutralizing antibodies target the Ace2 binding site of the spike, a subject showing low abundance or no SARS-CoV-2 spike protein and/or RBD specific and/or Ace2 binding site specific neutralizing antibodies is thus incapable of efficiently blocking spike binding to the Ace2 receptor.

The amount of specific antibodies may be determined by any method known to the skilled person, for example by lateral flow assay (LFA), ELISA, or preferably bead based multiplexed immunoassays. Antibody titers that can block the interaction between RBD and Ace2 may be determined by any method to the skilled person, for example by a blocking ELISA detection tool. The amount of neutralizing antibodies may be determined by any method known to the skilled person, for example by neutralization assays with real virus or pseudovirus. The sample may any one used in the art, preferably blood.

For human subjects medium-to-low abundance of SARS-CoV-2 neutralizing antibodies is defined as half maximum neutralizing titers below 1:500, low abundance of neutralizing antibodies is defined as half maximum neutralizing titers below 1:100 and very low abundance of neutralizing antibodies is defined as half maximum neutralizing titers below 1:50 measured at any time after seroconversion due to SARS-CoV-2 infection or vaccination.

For human subjects medium-to-low abundance of SARS-CoV-2 RBD specific IgG or IgA antibodies is defined as endpoint titers below 1:200 at any day in the first 10 days after symptom onset upon SARS-CoV-2 infection or after vaccination; low abundance of SARS-CoV-2 RBD specific IgG or IgA antibodies is defined as endpoint titers below 1:100 at any day in the first 10 days after symptom onset upon SARS-CoV-2 infection or after vaccination; very low abundance of SARS-CoV-2 RBD specific IgG or IgA antibodies is defined as endpoint titers below 1:50 at any day in the first 10 days after symptom onset upon SARS-CoV-2 infection or after vaccination. Medium-to-low abundance of SARS-CoV-2 RBD specific IgG or IgA antibodies is defined as endpoint titers below 1:10000 at any day post 10 days of symptom onset; low abundance of SARS-CoV-2 RBD specific IgG or IgA antibodies is defined as endpoint titers below 1:1000 at any day post 10 days of symptom onset; very low abundance of SARS-CoV-2 RBD specific IgG or IgA antibodies is defined as endpoint titers below 1:100 at any day in the first 10 days after symptom onset.

For human subjects medium-to-low Ace2 binding site specific antibodies are defined as below 50% inhibition of spike binding or RBD binding to Ace2 determined at a 1:20 serum dilution. Low Ace2 binding site specific antibodies are defined as below 25% inhibition of spike binding or RBD binding to Ace2 determined at a 1:20 serum dilution. Very low Ace2 binding site specific antibodies are defined as below 5% inhibition of spike binding or RBD binding to Ace2 determined at a 1:20 serum dilution.

In one preferred embodiment, the subject is a SARS-CoV-2 infected human person. More, preferably, the SARS-CoV-2 infected subject is positive in endonasal or throat swab.

A SARS-CoV-2 infected subject may be asymptomatic, or may have developed a mild disease, i.e. with mild symptoms or may have developed a severe disease, i.e. with severe symptoms.

Mild symptoms of COVID-19 are for example fever, chills, cough, fatigue, muscle or body aches, headache, loss of taste or smell, sore throat, congestion or runny nose, nausea, vomiting. Severe symptoms of COVID-19 are for example shortness of breath or difficulty breathing, pneumonia, ARDS, inflammatory symptoms, neurological manifestations, low blood-oxygen levels, hypoxia, low oxygen saturation, increased blood clotting. Symptoms usually appear 2-14 days after exposure to the virus.

The SARS-CoV-2 infected subject may be at an early stage of disease onset, or maybe already at late stage of disease onset. An early stage of COVID-19 is defined as the first 10 days after symptom onset.

In one preferred embodiment, the SARS-CoV-2 infected subject is asymptomatic, or has developed mild disease at an early stage of disease onset.

Accordingly, in such early stage of disease onset with no or mild symptoms the subject may specifically benefit from the ADAM17 inhibitor in supporting the SARS-CoV-2 induced antibody response.

In another preferred embodiment, the SARS-CoV-2 infected subject has developed severe symptoms of COVID-19, preferably inflammatory symptoms.

Inflammatory symptoms of COVID 19 in humans are typically moderately elevated C-reactive protein (CRP) at 10-60mg/l or elevated CRP at 60-200 mg/l, and highly elevated above 200 mg/l. Further inflammatory symptoms of COVID-19 are elevated IL6 at 35-80 pg/ml and highly elevated IL6 levels above 80 pg/ml.

Inflammatory symptoms in humans manifest in an increased amount of pro-inflammatory Angiotensin II (Angll) above 200 pg/ml and/or pro-inflammatory cytokines such as TNF-alpha above 10 pg/ml.

Preferably, the SARS-CoV-2 infected human subject shows an amount of Angll in serum or plasma of at least 200 pg/ml. Preferably, the SARS-CoV-2 infected subject shows an increased amount of Angll in serum or plasma compared to a healthy reference of at least 2x fold, more preferably of at least 3x fold, and most preferably at least 4x fold. The sample may be a serum or plasma sample deriving from the subject, more preferably, the sample is human serum or plasma.

The amount of soluble Angll may be determined by any method known to the skilled person, for example by ELISA, Western Blot.

Angll, which is the substrate of the Ace2 enzyme, has pro-inflammatory as well as strong vasoconstriction effects. The enzymatic product of Ace2, Angiotensin 1-7 (Ang1-7), by contrast, was shown to support the resolution of inflammation. Ace2 acts in a negative feedback interfering with the activity of the enzyme Ace, that converts Angiotensin I (Angl) to Angll (Yu et al., 2016). Coronavirus increases shedding of Ace2 and might also trap the enzyme by binding to the virus spike protein. Although binding of the SARS-CoV-1 spike protein to Ace2 has reported not to interfere with enzymatic activity (Li et al., 2005), through shedding, local concentrations of the active Ace2 enzyme in the lung might be lower leading to an accumulation of pro-inflammatory Angll. Interestingly, the H7N9 influenza virus, which does not use Ace2 as receptor, induced significant downregulation of Ace2. Also Angll levels were elevated in plasma samples of H7N9-infected individuals. Thus, it has been suggested that reduced Ace2 causes severe lung injury supported by the finding that Ace2 deficiency worsened disease pathogenesis (Yang et al., 2014). Under hypoxia, a lack of Ace2 may contribute to increased Angll thereby leading to vasoconstrictions in pulmonary circulation and low blood flow, which in turn causes ventilation perfusion mismatch. In a cohort study with 539 viral pneumonia patients, Ace1 inhibitor use was associated with an elevated risk of death or need for intubation but, interestingly, continued use during the hospital admission was possibly beneficial (Henry et al., 2018). A phase II clinical trial of human recombinant Ace2 (GSK2586881) in patients with acute respiratory distress syndrome was reported to be safe and to increase Ang1-7 but reduce Angll and IL6 in plasma (Khan et al., 2017). Hence, using an ADAM17 inhibitor that interferes with the release and local downregulation of Ace2 may provide anti-inflammatory effects and may reduce vasoconstriction effects in COVID-19 patients.

Preferably, the SARS-CoV-2 infected subject shows an amount of TNF-alpha in serum or plasma of at least 10 pg/ml. Preferably, the SARS-CoV-2 infected subject shows an increased amount of TNF-alpha in serum or plasma compared to a healthy reference of at least 1.5 fold, more preferably of at least 3 fold, and most preferably at least 4 fold. The sample may be a serum or plasma sample deriving from the subject, more preferably, the sample is human serum or plasma.

The amount of soluble TNF-alpha may be determined by any method known to the skilled person, for example by ELISA, Western Blot.

Many patients with severe COVID-19 disease display a so called cytokine storm by substantially elevated levels of pro-inflammatory cytokines in their serum, such as TNF-alpha (Wang et al., 2020; Huang et al., 2020). Viral pneumonia induced by respiratory syncytial virus (RSV) and influenza can be ameliorated by TNF-alpha blockade in preclinical mouse studies (Hussell et al., 2001). The ADAM17 protease was originally identified as the major enzyme for TNF-alpha release by cleaving membrane bound proTNF-alpha (Black et al., 1997; Moss et al., 1997). Inhibition of ADAM17 has been proposed to treat TNF-alpha mediated inflammatory diseases (reviewed in: (Arribas and Esselens, 2009; Moss and Minond, 2017). The use of an ADAM17 inhibitor thus has a beneficial anti-inflammatory effect for SARS-CoV-2 infected subjects and COVID-19 patients.

In Rheumatoid arthritis, TNF-alpha signaling is hyperactivated and contributes to chronic joint inflammation. ADAM17 mRNA expression is upregulated in arthritis-affected but not in normal cartilage (Patel et al., 1998). Interestingly, low oxygen conditions increase the expression of ADAM17 thereby leading to increased shedding of TNF-alpha in synoviocytes (Charbonneau et al., 2007). A selective inhibitor of ADAM17 (BMS-561392) has reached clinical Phase II and provided the proof of concept for beneficial effects in sepsis and rheumatoid arthritis, but the development was discontinued due to concerns over liver toxicity (Moss et al., 2008). While some metalloprotease inhibitors act not only on ADAM17 but also homologue proteases, inhibitory antibodies (MEDI3622, D1/A12, A9/B8) were developed that are highly selective for ADAM17 (Reviewed in: (Moss and Minond, 2017)). The D1/A12 antibody demonstrated suitable phar-macokinetics in mice and shedding of TGF-alpha was significantly reduced in plasma and even more prominent in ascitic fluid (by 15-fold). TNF-alpha was moderately reduced in ascitic fluid by 3.7-fold but not in plasma, suggesting compensation of shedding activity by other proteases (Richards et al., 2012). As the lack of species specific cross-reactivities hampered further clinical development of D1/A12, the A9/B8 antibody was developed and shown to attenuate Angll induced cardiovascular remodeling but not hypertension in mice (Takayanagi et al., 2016). In addition, another ADAM17 specific antibody MEDI3622 was shown to strongly reduce shedding of TNF-alpha in the mouse blood following injection of LPS (Rios-Doria et al., 2015). Clinical studies of ADAM17 inhibitors in rheumatoid arthritis have been discontinued but the current treatments (EMBREL, HUMIRA) are highly specific to TNF-alpha, while more than 80 substrates were described for ADAM17.

Hence, the ADAM17 inhibitor for use decreases vasoconstrictive effects and promotes an anti-inflammatory environment. Thereby, the ADAM17 inhibitor for use prevents or at least ameliorates the inflammatory and vasoconstrictive symptoms of COVID-19.

In a further preferred embodiment, the subject is of risk, preferably high risk to develop severe COVID-19 disease outcome upon infection with SARS-CoV-2.

Accordingly, the subject may be a non-infected or an infected subject. The subject may benefit from the ADAM17 inhibitor either upon natural infection or vaccination. Beside virus-induced shedding, elevated serum levels of soluble Ace2 were associated with diseases that were reported to be a risk factor for the development of severe COVID-19, for example cardiovascular disease and diabetes. For instance, in a cohort study of 228 subjects suspected of having clinical heart failure, soluble Ace2 activity strongly correlated with cardiovascular disease severity (Epelman et al., 2008). By normalizing enzyme activity to recombinant Ace2 standard curves, 13.7 +/- 3.6 ng/ml soluble Ace2 was detected in commercially available human plasma. In cohort subjects, 54,4% of heart failures (HF) were observed among individuals with Ace2 levels below 16.6ng/ml, 67.8% HF in the group of 16.6- 41.1ng/ml Ace2 and 89.3% HFs were found if serum levels were higher than 41.1ng/ml (p-value across quartiles 0.0002). In another family cohort study, soluble Ace2 was only detectable in 40 of 534 donors (Rice et al., 2006). Considering the calculated molecular weight of human Ace2 at 85kDa (Poglitsch et al., 2012), the enzyme concentrations detected by Rice et al. in Ace2 positive plasma ranged from 0,27ng/ml - 39.6 ng/ml (mean 2.8ng/ml). Importantly, Ace2 positive individuals tended to be older (p=0.002), had a higher waste to hip ratio (p=0.001), blood pressure (p <0.0005), fasting glucose levels (p <0.0005), cholesterol (p <0.0005) and triglycerides (p <0.0005). A later study showed that detection of Ace2 enzymatic activity in human plasma is masked by an endogenous inhibitor (Lew et al., 2008). By removing the inhibitory compound, the authors were able to detect Ace2 enzyme activity in any of 18 volunteer ranging between 1.31 to 8.69 pmol substrate cleaved min-1 ml-1. Thus, concentration of soluble Ace2 detected in previous studies might have been masked and could be even higher. Using the more sensitive assay, another study reported a median Ace2 activity of 29.3 pmol substrate cleaved min-1 ml-1 in 79 patients with obstructive coronary artery disease (Ramchand et al., 2018). Over a median follow up of 10.5 years, elevated plasma Ace2 activity was predictive for major adverse cardiovascular events (p = 0.009) and heart failure (p = 0.009). Using a less sensitive Ace2 human ELISA assay, 136 subjects undergoing coronary artery bypass grafting displayed around 1ng/ml soluble serum Ace2 levels pre- and around 4 ng/ml 6 days post-surgery (Wang et al., 2014). Other studies highlighted the correlation between higher levels of circulating Ace2 and systolic dysfunction in human hypertension and heart failure (Uri et al., 2014), cardiovascular disease development (Uri et al., 2016) acute heart failure requiring emergency hospitalization (Hisatake et al., 2017), cardiovascular risk factors like age, diabetes and male gender in a chronic kidney disease cohort (Anguiano et al., 2015). In addition, elevated urinary Ace2 levels were detected in renal transplant patients with diabetes (Xiao et al., 2012) and type 2 diabetic mellitus (Liang et al., 2015). In a type 2 diabetic mouse model, daily exercise training significantly lowered Ace2 excretion and was thus suggested as a prognostic tool for kidney damage progression (Somineni et al., 2014).

ADAM17 as well as Ace2 mRNA has been detected in mouse muscles and adipose tissue, and Ace2 is even upregulated in muscles of diabetic mice (Pedersen et al., 2015). The human protein atlas not only confirms Ace2 mRNA expression in adipose tissue, very high protein expression scores were also reported for male tissues such as the testis (The Human Protein Atlas). And in mice, Ace2 activity in the male kidney was significantly higher than in female kidney tissue (ACE2 activity male 18.1 +/- 1.0 versus female 11.1 +/- 0.39 0.39 (RFU/min/µg protein), P < 0.0001) (Liu et al., 2010). These sex related differences have been attributed to sex hormones (Hilliard et al., 2013). COVID-19 disease severity and death is strongly gender associated. Although men and women get equally infected by the virus, the death rate in male is >60% in the majority of countries (globalhealth5050). In Italy for instance, 67% of death were accounted to male by the 9^{th} of April 2020.

Soluble Ace2 may mask SARS-CoV-2 spike protein thereby shielding crucial epitopes from induction of neutralizing antibodies, consequently, increased levels of soluble Ace2 interfere with the generation of antibodies that block virus uptake. Consequently, subjects with elevated soluble Ace2 levels such as patients with coronary heart disease and diabetes as well as healthy individuals with coronary risk factors or other risk factors as discussed may in turn have less effective antibody responses to SARS-CoV-1 and, due to an increased receptor affinity, even more poor responses to SARS-CoV-2.

Accordingly, a subject of risk to develop severe COVID-19 disease outcome is a subject with comorbidities associated with increased soluble Ace2 levels, and/or an increased amount or concentration of Ace2, and/or an increased Ace2 mRNA expression, and/or an genetic predisposition for high Ace2 receptor expression, and/or an genetic predisposition for high Ace2 receptor shedding, and/or low abundance or no neutralizing antibodies, and/or suffers from a condition or disease associated with elevated serum levels of soluble Ace2.

Individuals are at risk to develop severe COVID-19 when they are obese, male, smoker, suffering from hypertension, coronary heart disease, kidney disease or type 2 diabetes (T2D). Individuals are at high risk when displaying at least two of these parameters.

Preferably, the subject shows an increased amount of soluble Ace2 in serum or plasma compared to a reference of at least 1.5 fold, more preferably of at least 3 fold, more preferably at least 5 fold and most preferably at least 10 fold. The reference is the median values of a cohort of mean age of 30-50 years, without comorbidities associated with COVID-19. The sample may be a serum or plasma sample deriving from the subject, more preferably, the sample is human serum or plasma.

The amount of soluble Ace2 may be determined by any method known to the skilled person, for example by ELISA, Western Blot, or, most preferably, Ace2 enzymatic activity measurement.

Preferably, the subject shows a concentration of soluble Ace2 in human serum or plasma of at least 1 ng/ ml, preferably at least 10 ng/ml, preferably at least 16 ng/ml, more preferably at least 41 ng/ml determined by enzymatic activity measurement. Most preferably, the subject shows a concentration of at least 50 ng/ml soluble Ace2 in human serum or plasma determined by enzymatic activity measurement.

Preferably, the subject shows an increased Ace2 mRNA expression compared to a reference of at least 1.5 fold, most preferably at least 3 fold. The reference is the median values of a cohort of mean age of 30-50 years, without comorbidities associated with COVID-19. The sample may be any cell sample deriving from the subject, more preferably a human cell sample, most preferably, the sample is a human mucosal biopsy.

Ace2 mRNA expression may be determined by any method known to the skilled person, for example by RT-PCR or RNA-Sequencing.

Preferably, the subject has a genetic predisposition for high Ace2 receptor expression or Ace2 receptor shedding. In a Chinese population three Ace2 variants were reported to be associated with hypertension (rs4240157, rs4646155, rs4830542) (Yi et al, 2006; Niu et al, 2007; Fan et al., 2009; Chen et al, 2016; Luo et al., 2019). A canadian study cohort for nicotine dependence revealed three mutations (rs2074192, rs233575, rs2158083) that were associated with pathological variations of blood pressure (Malard et al. 2013). Another Ace2 mutation was associated with clinical manifestations of hypertension (rs21068809) (Chen et al. 2010). A study with 246 patients with hypertension and 274 normo-tensive people conducted in India revealed an association of hypertension with an Ace2 mutation (rs21068809) (Patnaik et al, 2014). A Brazilian study suggested a contribution of an Ace2 polymorphism (G8790A) to hypertension. Pref-ereably, the genetic predisposition is selected from risk genotype rs4240157, rs4646155, rs4830542, rs2074192, rs233575, rs2158083, rs21068809.

Genetic predisposition for high Ace2 receptor expression or Ace2 receptor shedding may be determined by any method known to the skilled person, for example by genomic PCR or reverse transcriptase cDNA amplification from Ace2-RNA followed by sequencing or restriction fragment length polymorphism (RFLP) analysis. The sample may be any cell sample deriving from the subject, more preferably a human cell sample.

Preferably, the subject suffers from a condition or disease selected from cardiovascular disease, diabetes, obstructive coronary artery disease, systolic dysfunction in human hypertension and heart failure, high age of at least 60 years, male, waste to hip ratio of at least 0.87 cm, obesity with a BMI of at least 34.7 kg/m², a systolic blood pressure of at least 134.7 mm Hg, a faster glucose level of at least 5.24 mmol/L, a concentration of cholesterol of at least 5.26 mmol/L, a concentration of triglycerides of at least 1.54 mmol/L.

An ADAM17 inhibitor is defined as a compound which represses the metalloproteinase "sheddase" activities of ADAM17. That is an ADAM17 inhibitor prevents cleavage of ACE2 and thereby the release of soluble Ace2. In the present invention, it is to be understood that any preferred embodiment disclosed in the following for the ADAM17 inhibitor is applicable to any embodiment as disclosed above with respect to the SARS-CoV-2 infected and non-infected subjects including the subjects at risk.

Examples of an ADAM17 inhibitor are antibodies, siRNAs, or chemical compounds. Preferably, the ADAM17 inhibitor is a chemical compound.

More preferably, the ADAM17 inhibitor is a chemical compound selected from methyl (6*S*,7*S*)-7-(hydroxycarbamoyl)-6-(4-phenylpiperazine-1-carbonyl)-5-azaspiro[2.5]octane-5-carboxylate ("Aderbasib", codenamed INCB7839, CAS Number: 791828-58-5); methyl (5S,6S)-5-(hydroxycarbamoyl)-6-(4-phenyl3,6-dihydro-2H-pyridine-1-carbonyl)-7-azaspiro[2.5]octane-7-carboxylate (codenamed: INCB3619;, CAS number 791826-72-7); (3S)-N-Hydroxy-4-((4-((4-hydroxybut-2-ynyl)oxy)phenyl)sulfonyl)-2,2-dimethylthiomorpholine-3-carboxamide(3S)-N-Hydroxy-4-((4-((4-hydroxybut-2-ynyl)oxy)phenyl)sulfonyl)-2,2-dimethylthiomorpholine-3-carboxamide ("Apratastat", codenamed: TMI005; XMT1191, CAS Number: 287405-51-0); (3S)-4-[[4-(2-Butyn-1-yloxy)phenyl]sulfonyl]-N-hydroxy-2,2-dimethyl-3-thiomorpholinecarboxamide (codenamed: TMI-1, CAS Number: 287403-39-8), Acetamide, 2-(2,2-dimethyl-5-oxo-1,3-dioxolan-4-ylidene)-N-((4-fluorophenyl)methyl)-N-methoxy-(codenamed :; BMS 561392; DPC333., CAS Number: 611227-74-8); (2R)-N-hydroxy-2-[(3S)-3-methyl-3-[4-[(2-methylquinolin-4-yl)methoxy]phenyl]-2-oxopyrrolidin-1-yl]propanamide (codenamed: IK862; CAS Number: 478911-60-3);(2R)-5-[(Aminocarbonyl)amino]-2-[[[4-[(3,5-dibromophenyl)methoxy]phenyl]sulfonyl]amino]-N-hydroxy-pentanamide (codenamed: JG26; CAS Number: 1464910-32-4); (2S,3R)-2-cinnamyl-N-hydroxy-3-(2-isobutyl-2-(methylsulfonyl)hydrazine-1-carbonyl)-5-methylhexanamide (codenamed: Ro327315, CAS Number: 219613-02-2) ; N-[(2S)-1-[[(2S)-1-(2-Aminoethylamino)-1-oxopropan-2-yl]amino]-3-naphthalen-2-yl-1-oxopropan-2-yl]-N'-hydroxy-2-(2-methylpropyl)butanediamide (codenamed: TAPI-1, TNF-alpha Protease Inhibitor-1; CAS Number: 171235-71-5); (3R,4R)-N-Hydroxy-4-[[4-[[2-(trifluoromethyl)benzimidazol-1-yl]methyl]benzoyl]amino]oxane-3-carboxamide (codenamed: BMS566394,; CAS Number: 503166-51-6); (R)-N1-((S)-3-(1H-indol-3-yl)-1-(methylamino)-1-oxopropan-2-yl)-N4-hydroxy-2-isobutylsuccinamide ("Ilomastat"; codenamed: GM6001, , galardin; CAS Number: 142880-36-2); (2R,3S)-3-(Formyl-hydroxyamino)-2-(2-methyl-1-propyl)-4-methylpentanoic acid, ((1S,2S)-2-methyl-1-(2-pyridylcar-bamoyl)-1-butyl)amide (codenamed: GW3333; CAS Number: 212609-68-2); (2S,3S)-3-N-hydroxy-1-methyl-2-N-[4-[(2-methylquinolin-4-yl)methoxy]phenyl]piperidine-2,3-dicarboxamide (codenamed: IM-491, UNII-UG82Y9C069, CHEMBL134908, SCHEMBL6444631, UG82Y9C069, DNC014796 ; CAS Number: 252918-63-1); (2S,3R)-N1-Hydroxy-3-isobutyl-2-methyl-N4-((S)-1-(methyl-amino)-1-oxo-3-phenylpropan-2-yl)succinamide (codenamed: KBR7785; CAS Number: 168158-16-5); N-(R)-[2-(Hydroxyaminocarbonyl)methyl]-4-methylpentanoyl-L-t-butyl-alanyl-L-alanine, 2-aminoethyl Amide (codenamed: TAPI2; CAS Number: 187034-31-7); (2S,3R)-N4-((S)-2,2-Dimethyl-1-methylcarbamoyl-propyl)-N1-hydroxy-2-hydroxymethyl-3-(4-methoxy-phenyl)-succinamide) (codenamed: PKF242-484); (2S,3R)-N4-((S)-2,2-Dimethyl-1-methylcarbamoyl-propyl)-N1-hydroxy-2-hydroxymethyl-3-phenyl-succinamide (codenamed: PKF241-466); N-(2-{[4-(but-2-yn-1-yloxy)phenyl]sulfonyl}-1-[4-(methylsulfonamidomethyl)phenyl]ethyl)-N-hydroxyformamide (codenamed: KP-457); 1-[4-(4-fluoro-2-methylbenzyloxy)-benzenesulfonyl]-3-hydroxy-3-methylpiperidine-2-carboxylic acid hydroxyamide (codenamed: CP-661,631); N-{4_-[2-(hydroxylamino)-2-oxoethyl]-2_,6_-dimethyl-4-piperidinyl}-4-[(2-methyl-4-quinolinyl)methoxy]benzamide (codenamed: DPH-067517); Benzyl N-[(5S)-5-[[(2R,3S)-3-[formyl(hydroxy)amino]-2-(2-methylpropyl)hexanoyl]amino]-6-oxo-6-(1,3-thiazol-2-ylamino)hexyl]carbamate (codenamed: GW280264X, CAS Number: 866924-39-2,); (2R,3S)-N-[(2S)-5-[[amino(nitramido)methylidene]amino]-1-oxo-1-(1,3-thiazol-2-ylamino)pentan-2-yl]-3-[formyl(hydroxy)amino]-2-(2-methylpropyl)hexanamide (codenamed: GW4459,); 2R)-N-Hydroxy-2-[(3S)-3-methyl-4-[4-[(2-methylquinolin-4-yl)methoxy]phenyl]-2-oxopyrrolidin-1-yl]propanamide (codenamed: IK682); compounds codenamed WTACE2, XL784; BB3103, TMI-2, R-618, INCB4298, DPC-A38088; GI5402 (GI-245402, BB-2983).

Even more preferably, the ADAM17 inhibitor is a compound selected from methyl (6*S*,7*S*)-7-(hydroxycarbamoyl)-6-(4-phenylpiperazine-1-carbonyl)-5-azaspiro[2.5]octane-5-carboxylate ("Aderbasib", codenamed: INCB7839, CAS Number: 791828-58-5); methyl (5S,6S)-5-(hydroxycarbamoyl)-6-(4-phenyl3,6-dihydro-2H-pyridine-1-carbonyl)-7-azaspiro[2.5]octane-7-carboxylate (codenamed: INCB3619;, CAS number 791826-72-7); (3S)-N-Hydroxy-4-((4-((4-hydroxybut-2-ynyl)oxy)phenyl)sulfonyl)-2,2-dimethylthiomorpholine-3-carboxamide(3S)-N-Hydroxy-4-((4-((4-hydroxybut-2-ynyl)oxy)phenyl)sulfonyl)-2,2-dimethylthiomorpholine-3-carboxamide ("Apratastat", codenamed: TMI005; XMT1191, CAS Number: 287405-51-0); (3S)-4-[[4-(2-Butyn-1-yloxy)phenyl]sulfonyl]-N-hydroxy-2,2-dimethyl-3-thiomorpholinecarboxamide (codenamed: TMI-1, CAS Number: 287403-39-8), Acetamide, 2-(2,2-dimethyl-5-oxo-1,3-dioxolan-4-ylidene)-N-((4-fluorophenyl)methyl)-N-methoxy-(codenamed :; BMS 561392; DPC333., CAS Number: 611227-74-8); (2R)-N-hydroxy-2-[(3S)-3-methyl-3-[4-[(2-methylquinolin-4-yl)methoxy]phenyl]-2-oxopyrrolidin-1-yl]propanamide (codenamed: IK862; CAS Number: 478911-60-3);(2R)-5-[(Aminocarbonyl)amino]-2-[[[4-[(3,5-dibromophenyl)methoxy]phenyl]sulfonyl]amino]-N-hydroxy-pentanamide (codenamed: JG26; CAS Number: 1464910-32-4); (2S,3R)-2-cinnamyl-N-hydroxy-3-(2-isobutyl-2-(methylsulfonyl)hydrazine-1-carbonyl)-5-methylhexanamide (codenamed: Ro327315, CAS Number: 219613-02-2) ; N-[(2S)-1-[[(2S)-1-(2-Aminoethylamino)-1-oxopropan-2-yl]amino]-3-naphthalen-2-yl-1-oxopropan-2-yl]-N'-hydroxy-2-(2-methylpropyl)butanediamide (codenamed: TAPI-1, TNF-alpha Protease Inhibitor-1; CAS Number: 171235-71-5); (3R,4R)-N-Hydroxy-4-[[4-[[2-(trifluoromethyl)benzimidazol-1-yl]methyl]benzoyl]amino]oxane-3-carboxamide (codenamed: BMS566394,; CAS Number: 503166-51-6); (R)-N1-((S)-3-(1H-indol-3-yl)-1-(methylamino)-1-oxopropan-2-yl)-N4-hydroxy-2-isobutylsuccinamide ("Ilomastat"; codenamed: GM6001, , galardin; CAS Number: 142880-36-2); (2R,3S)-3-(Formyl-hydroxyamino)-2-(2-methyl-1-propyl)-4-methylpentanoic acid, ((1S,2S)-2-methyl-1-(2-pyridylcarbamoyl)-1-butyl)amide (codenamed: GW3333; CAS Number: 212609-68-2); (2S,3S)-3-N-hydroxy-1-methyl-2-N-[4-[(2-methylquinolin-4-yl)methoxy]phenyl]piperidine-2,3-dicarboxamide (codenamed: IM-491, UNII-UG82Y9C069, CHEMBL134908, SCHEMBL6444631, UG82Y9C069, DNC014796 ; CAS Number: 252918-63-1); (2S,3R)-N1-Hydroxy-3-isobutyl-2-methyl-N4-((S)-1-(methyl-amino)-1-oxo-3-phenylpropan-2-yl)succinamide (codenamed: KBR7785; CAS Number: 168158-16-5); N-(R)-[2-(Hydroxyaminocarbonyl)methyl]-4-methylpentanoyl-L-t-butyl-alanyl-L-alanine, 2-aminoethyl Amide (codenamed: TAPI2; CAS Number: 187034-31-7).

Even more preferably, the ADAM17 inhibitor is a chemical compound selected from methyl (6*S*,7*S*)-7-(hydroxycarbamoyl)-6-(4-phenylpiperazine-1-carbonyl)-5-azaspiro[2.5]octane-5-carboxylate ("Aderbasib", codenamed: INCB7839, CAS Number: 791828-58-5); methyl (5S,6S)-5-(hydroxycarbamoyl)-6-(4-phenyl3,6-dihydro-2H-pyridine-1-carbonyl)-7-azaspiro[2.5]octane-7-carboxylate (codenamed: INCB3619;, CAS number 791826-72-7); (3S)-N-Hydroxy-4-((4-((4-hydroxybut-2-ynyl)oxy)phenyl)sulfonyl)-2,2-dimethylthiomorpholine-3-carboxamide(3S)-N-Hydroxy-4-((4-((4-hydroxybut-2-ynyl)oxy)phenyl)sulfonyl)-2,2-dimethylthiomorpholine-3-carboxamide ("Apratastat", codenamed: TMI005; XMT1191, CAS Number: 287405-51-0); (3S)-4-[[4-(2-Butyn-1-yloxy)phenyl]sulfonyl]-N-hydroxy-2,2-dimethyl-3-thiomorpholinecarboxamide (codenamed: TMI-1, CAS Number: 287403-39-8), Acetamide, 2-(2,2-dimethyl-5-oxo-1,3-dioxolan-4-ylidene)-N-((4-fluorophenyl)methyl)-N-methoxy-(codenamed :; BMS 561392; DPC333., CAS Number: 611227-74-8); (2R)-N-hydroxy-2-[(3S)-3-methyl-3-[4-[(2-methylquinolin-4-yl)methoxy]phenyl]-2-oxopyrrolidin-1-yl]propanamide (codenamed: IK862; CAS Number: 478911-60-3);(2R)-5-[(Aminocarbonyl)amino]-2-[[[4-[(3,5-dibromophenyl)methoxy]phenyl]sulfonyl]amino]-N-hydroxy-pentanamide (codenamed: JG26; CAS Number: 1464910-32-4); (2S,3R)-2-cinnamyl-N-hydroxy-3-(2-isobutyl-2-(methylsulfonyl)hydrazine-1-carbonyl)-5-methylhexanamide (codenamed: Ro327315, CAS Number: 219613-02-2) ; N-[(2S)-1-[[(2S)-1-(2-Aminoethylamino)-1-oxopropan-2-yl]amino]-3-naphthalen-2-yl-1-oxopropan-2-yl]-N'-hydroxy-2-(2-methylpropyl)butanediamide (codenamed: TAPI-1, TNF-alpha Protease Inhibitor-1; CAS Number: 171235-71-5)

Even more preferably, the ADAM17 inhibitor is a chemical compound selected from methyl (6*S*,7*S*)-7-(hydroxycarbamoyl)-6-(4-phenylpiperazine-1-carbonyl)-5-azaspiro[2.5]octane-5-carboxylate ("Aderbasib", codenamed: INCB7839, CAS Number: 791828-58-5); methyl (5S,6S)-5-(hydroxycarbamoyl)-6-(4-phenyl3,6-dihydro-2H-pyridine-1-carbonyl)-7-azaspiro[2.5]octane-7-carboxylate (codenamed: INCB3619;, CAS number 791826-72-7); (3S)-N-Hydroxy-4-((4-((4-hydroxybut-2-ynyl)oxy)phenyl)sulfonyl)-2,2-dimethylthiomorpholine-3-carboxamide(3S)-N-Hydroxy-4-((4-((4-hydroxybut-2-ynyl)oxy)phenyl)sulfonyl)-2,2-dimethylthiomorpholine-3-carboxamide ("Apratastat", codenamed: TMI005; XMT1191, CAS Number: 287405-51-0); (3S)-4-[[4-(2-Butyn-1-yloxy)phenyl]sulfonyl]-N-hydroxy-2,2-dimethyl-3-thiomorpholinecarboxamide (codenamed: TMI-1, CAS Number: 287403-39-8), Acetamide, 2-(2,2-dimethyl-5-oxo-1,3-dioxolan-4-ylidene)-N-((4-fluorophenyl)methyl)-N-methoxy-(codenamed: BMS 561392; DPC333., CAS Number: 611227-74-8).

Most preferably, the ADAM17 inhibitor is methyl (6S,7S)-7-(hydroxycarbamoyl)-6-(4-phenylpiperazine-1-carbonyl)-5-azaspiro[2.5]octane-5-carboxylate ("Aderbasib", codenamed INCB7839, CAS Number: 791828-58-5).

The ADAM17 inhibitor may be administered to a SARS-CoV-2 infected subject at any time after SARS-CoV-2 infection. The median time of seroconversion in COVID-19 is 12 days for IgM and 14 days for IgG responses (Zhao et al., 2020). The mean time from onset to death has been estimated 17-18 days (Verity et al., 2020). While in survivors, the median duration of virus shedding was about 20 days (with 37 at longest), in non-survivors SARS-CoV virus was detectable until death (Zhou et al., 2020).

Hence, the ADAM17 inhibitor is administered starting as early as possible after SARS-CoV-2 infection. Preferably, the ADAM17 inhibitor is administered starting at the day of confirmed SARS-CoV-2 infection, i.e. day 1 to day 14 after confirmed SARS-CoV-2 infection, more preferably starting between days 1 to 7 after confirmed SARS-CoV-2 infection, most preferred at the day of confirmed SARS-CoV-2 infection.

The ADAM17 inhibitor may be administered to a SARS-CoV-2 non-infected subject as a prophylactic treatment. Preferably, such prophylactic treatment is for a subject of risk to develop severe COVID-19 disease outcome as defined above. Such prophylactic treatment is further preferable for a subject who is exposed to SARS-CoV-2 infected subjects and COVID-19 patients, for example subjects working in hospital facilities including nurses and medical practitioners.

In a further embodiment, the ADAM17 inhibitor is used in combination with a vaccine. The vaccine may be any vaccine and preferably is a vaccine for active immunization. Thus, preferably the vaccine does not comprise vaccines for passive immunization such as antibodies. More preferably, the vaccine is a recombinant protein, a Modified Vaccinia Virus Ankara vaccine, an inactivated pathogen, a DNA vaccine, or an RNA vaccine. Most preferably, the vaccine contains recombinant spike or RBD proteins or spike or RBD encoding nucleic acids.

The ADAM17, ADAM10 dual inhibitor methyl (6S,7S)-7-(hydroxycarbamoyl)-6-[(4-phenyl-1-piperazinyl)carbonyl]-5-azaspiro[2.5]octane-5-carboxylate (INCB7839) has been applied in a clinical trial of women with HER2+ metastatic breast cancer in combination with the HER specific antibody trastuzumab (Newton et al, 2016). INCB7839 markedly reduced HER2 cleavage and the clinical response rate. The combinations of applied drugs in the trial were reported generally safe and well tolerated. A completed clinical trial was applying INCB7839 together with Rituximab in diffuse large B cell lymphoma (NCT02141451). Another multicenter phase I trial of INCB7839 single compound treatment in progressive high grade gliomas (NCT04295759) has been posted on the 4th of March 2020 on clinicaltrials.gov. In the clinical trials for solid tumors and breast cancer, adverse effects of INCB7839 were mild to moderate in severity. The dose limiting toxicity was declared to be deep venous thrombosis (9/41 patients). Pro-coagulant effects were observed in some adult patients.

As the spike protein and its receptor binding domain are the main targets of SARS-CoV-2 vaccination strategies, shielding via soluble Ace2 may also occur during active immunization. To reduce adverse local effects, vaccines containing adjuvants are mainly administered intramuscular. ADAM17 as well as Ace2 mRNA has been detected in mouse muscles and adipose tissue, and Ace2 is even upregulated in muscles of diabetic mice (Pedersen et al., 2015). Therefore, recombinant proteins of vaccines may also be shielded by local Ace2. Hence, the use of ADAM17 inhibitor together with the vaccine may improve vaccination.

The ADAM17 inhibitor may be administered with the vaccine in any sequence. The ADAM17 inhibitor may be administered simultaneously with the vaccine but also before or after the vaccine is administered. Hence, the ADAM17 inhibitor may be administered for the first time starting at the day of vaccination. Preferably, the ADAM17 inhibitor is administered already prior to vaccination. Thereby Ace2 receptor shedding may be reduced before the vaccine is administered. More preferably, the ADAM17 inhibitor is administered one to seven days prior to vaccination, more preferably one to five days prior to vaccination more preferably three days prior to vaccination, most preferably two days prior to vaccination.

Repeated administration of ADAM17 inhibitor together with the vaccine for more than one time of up to four times may become necessary. Usually, vaccines are adminstered two to three times and accordingly the ADAM17 inhibitor is preferably administered two to three times together with the vaccine as defined above. Vaccination may need to be refreshed after several years or annually and accordingly, the ADAM17 inhibitor is again administered together with the vaccine as defined above.

Both for use in natural infection as well as vaccination, the ADAM17 inhibitor may be administered for one or more times a day for several consecutive days.

Preferably, the ADAM17 inhibitor is administered at least once a day for 10 to 40 consecutive days, preferably 14 to 25 consecutive days, more preferably 21 consecutive days. It is to be understood that the first application of ADAM17 inhibitor depends on whether it is used for natural infection or vaccination as defined above. Accordingly, the ADAM17 inhibitor is administered at least once a day for 10 to 40 consecutive days, preferably 14 to 25 consecutive days, more preferably 21 consecutive days after the first administration as defined above for natural infection as well as vaccination. Accordingly, in case of a natural infection the ADAM17 inhibitor is preferably administered at least once a day for 10 to 40 consecutive days preferably starting at the day of confirmed SARS-CoV-2 infection to day 14 after confirmed SARS-CoV-2 infection as defined above. Accordingly, in case of a vaccination the ADAM17 inhibitor is administered at least once a day for 10 to 40 consecutive days preferably starting already prior to vaccination.

The ADAM17 inhibitor may be administered once a day, to four times a day. The total dose per day may be 50 to 800 mg/m². Preferably, the ADAM17 inhibitor is administered twice a day with a single dose of 50 to 400 mg/m², more preferably twice a day at a dose of 80 to 200 mg/m².

The ADAM17 inhibitor as used in the present invention may be administered by any suitable route of administration. Preferably, the ADAM17 inhibitor is administered by systemic administration. Systemic administration includes oral administration, parenteral administration, transdermal administration, rectal administration, and administration by inhalation. Parenteral administration refers to routes of administration other than enteral, transdermal, or by inhalation, and is typically by injection or infusion. Parenteral administration includes intravenous, intramuscular, and subcutaneous injection or infusion. Inhalation refers to administration into the patient's lungs whether inhaled through the mouth or through the nasal passages. Preferably, the ADAM17 inhibitor is administered orally.

The ADAM17 inhibitors are usually formulated into pharmaceutical compositions. Hence, in a further aspect, the invention relates to a pharmaceutical composition comprising an ADAM17 inhibitor as an active ingredient for use in the prevention and/or treatment of COVID-19 in a subject. Further, pharmaceutically acceptable excipient meaning a pharmaceutically acceptable material, composition or vehicle involved in giving form or consistency to the pharmaceutical composition may be present in the pharmaceutical compositions.

The pharmaceutical compositions may optionally further comprise one or more additional pharmaceutically active compounds.

Preferably, the pharmaceutical compositions may further comprise a vaccine as defined above. Accordingly, the pharmaceutical composition comprises an ADAM17 inhibitor as an active ingredient and a vaccine for use in the prevention and/or treatment of COVID-19.

In a further aspect, the invention relates to a kit of parts for use in the prevention and/or treatment of COVID-19 in a subject comprising i) an ADAM17 inhibitor and ii) a vaccine.

The components for a kit of parts of the invention may be formulated for simultaneous administration or for administration in any sequence. The components may also be for repeated administration.

In a further aspect, the invention relates to a pharmaceutical composition comprising an ADAM17 inhibitor and active immunization vaccine.

In a further aspect, the invention relates to a kit of parts comprising i) an ADAM17 inhibitor and ii) an active immunization vaccine.

For the pharmaceutical composition and kit of parts the ADAM17 inhibitor and active immunization vaccine are defined above. More preferably, the vaccine is a recombinant protein, a Modified Vaccinia Virus Ankara vaccine, an inactivated pathogen, a DNA vaccine, or a RNA vaccine. The vaccine does not comprise vaccines for passive immunization such as antibodies.

In a further aspect, the invention relates to a method of preventing and/or treating COVID-19 in a subject, the method comprising administering a therapeutically effective amount of an ADAM17 inhibitor to a subject.

Preferably, the subject is in need of such prevention and/or treatment. Preferably, the subject is a mammal, more preferably a human in the need thereof.

In one preferred embodiment the subject is a non-infected subject. In another embodiment, the subject is a SARS-CoV-2 infected subject, i.e. a patient.

In the present invention, it is to be understood that any embodiment disclosed for the ADAM17 inhibitors including the compounds as well as administration schemes and/or dosage and any embodiment disclosed for the SARS-CoV-2 infected and non-infected subjects including the subjects at risk is applicable for either the pharmaceutical composition comprising an ADAM17 inhibitor as an active ingredient for use in the prevention and/or treatment of COVID-19, the kit of parts for use in the prevention and/or treatment of COVID-19 comprising i) an ADAM17 inhibitor and ii) a vaccine, the pharmaceutical composition comprising an ADAM17 inhibitor and active immunization vaccine, the kit of parts comprising i) an ADAM17 inhibitor and ii) a active immunization vaccine and the method of preventing and/or treating COVID-19 in a subject.

The invention is further illustrated by the following figures.

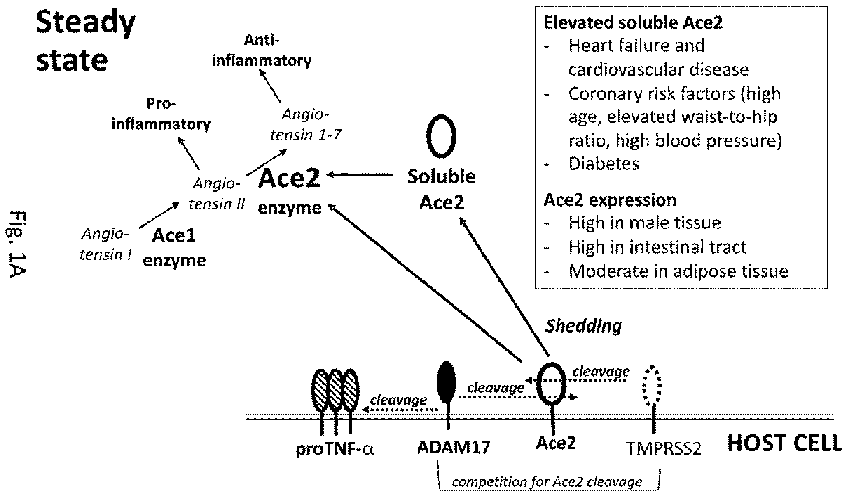

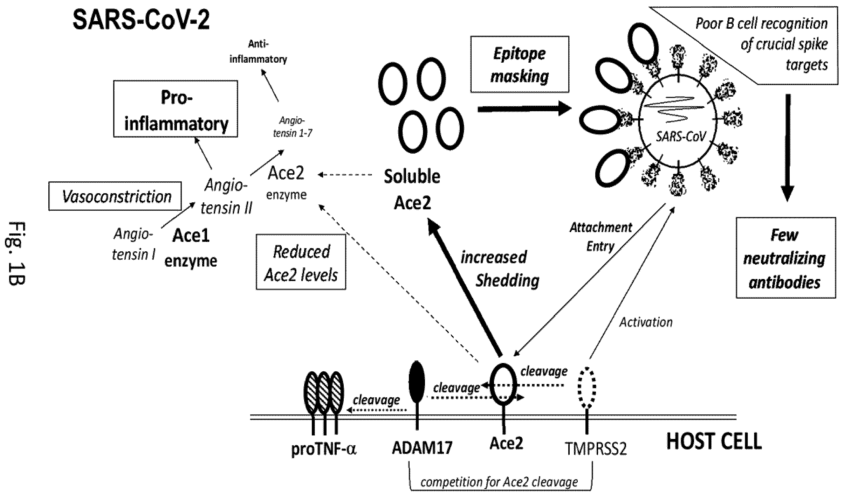

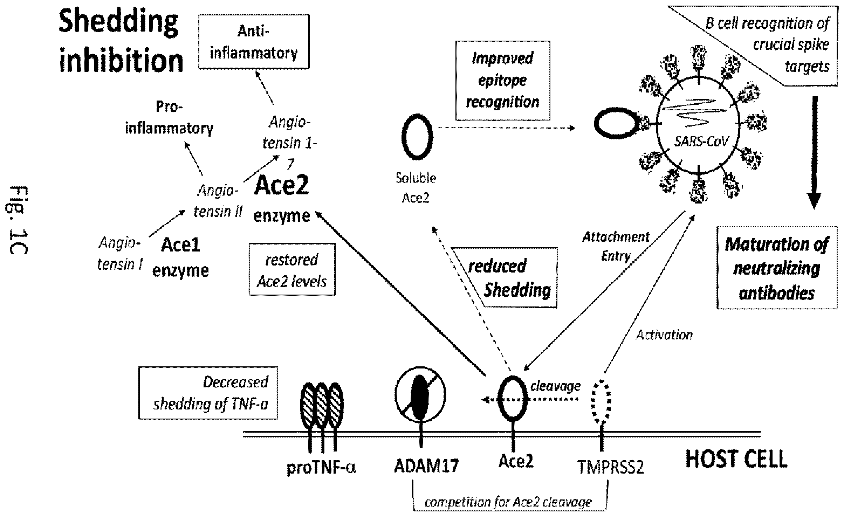

Figure 1: Ace2 shedding drives epitope masking, proinflammatory response and vasoconstriction during SARS-CoV-2 infection that may be ameliorated by ADAM17 inhibition. 1A) The scheme depicts Ace2-shedding under steady state and lists risk factors for higher soluble Ace2 levels. 1B) SARS-CoV-2 infection induces Ace2 shedding enabling epitope masking. Reduced Ace2 levels entail decreased enzymatic activity driving proinflammatory responses and vasoconstriction. 1C) Shedding inhibition by ADAM17 inhibitors could improve spike recognition and B cell activation and foster anti-inflammatory immune responses.

### LITERATURE

Althoff, K., P. Reddy, N. Voltz, S. Rose-John, and J. Müllberg. 2000. Shedding of interleukin-6 receptor and tumor necrosis factor alpha. Contribution of the stalk sequence to the cleavage pattern of transmembrane proteins. Eur. J. Bio-chem. 267:2624-2631. doi:10.1046/j.1432-1327.2000.01278.x.
Amanat, F., and F. Krammer. 2020. SARS-CoV-2 Vaccines: Status Report. Immunity. 52:583-589. doi:10.1016/j.immuni.2020.03.007.
Anguiano, L., M. Riera, J. Pascual, J.M. Valdivielso, C. Barrios, A. Betriu, S. Mojal, E. Fernández, M.J. Soler, NEFRONA study. 2015. Circulating angiotensin-converting enzyme 2 activity in patients with chronic kidney disease without previous history of cardiovascular disease. Nephrol. Dial. Transplant. 30:1176-1185. doi:10.1093/ndt/gfv025.
Arribas, J., and C. Esselens. 2009. ADAM17 as a therapeutic target in multiple diseases. Curr. Pharm. Des. 15:2319-2335. doi:10.2174/138161209788682398.
Bergström, J.J.E., and B. Heyman. 2015. IgG Suppresses Antibody Responses in Mice Lacking C1q, C3, Complement Receptors 1 and 2, or IgG Fc-Receptors. PLoS ONE. 10:e0143841. doi:10.1371/journal.pone.0143841.
Bergström, J.J.E., H. Xu, and B. Heyman. 2017. Epitope-Specific Suppression of IgG Responses by Passively Administered Specific IgG: Evidence of Epitope Masking. Frontiers in Immunology. 8:238. doi:10.3389/fimmu.2017.00238.
Bernardo, L., H. Yu, A. Amash, J.C. Zimring, and A.H. Lazarus. 2015. IgG-Mediated Immune Suppression to Erythrocytes by Polyclonal Antibodies Can Occur in the Absence of Activating or Inhibitory Fcγ Receptors in a Full Mouse Model. J. Immunol. 195:2224-2230. doi:10.4049/jimmunol.1500790.
Bicheng Zhang, Xiaoyang Zhou, Chengliang Zhu, Fan Feng, Yanru Qiu, Jia Feng, Qingzhu Jia, Qibin Song, Bo Zhu. 2020. Immune phenotyping based on neutrophil-to-lymphocyte ratio and IgG predicts disease severity and outcome for patients with COVID-19. medRxiv. Preprint. doi: https://doi.org/10.1101/2020.03.12.20035048
Bin Ju, Qi Zhang, Xiangyang Ge, Ruoke Wang, Jiazhen Yu, Sisi Shan, Bing Zhou, Shuo Song, Xian Tang, Jinfang Yu, Jiwan Ge, J un Lan, Jing Yuan, Haiyan Wang, Juanjuan Zhao, Shuye Zhang, Youchun Wang, Xuanling Shi, Lei Liu, Xin-quan Wang, Zh eng Zhang, View ORCID ProfileLinqi Zhang. 2020. Potent human neutralizing antibodies elicited by SARS-CoV-2 infection. medRxiv. Preprint. doi: https://doi.org/10.1101/2020.03.21.990770
Black, R.A., C.T. Rauch, C.J. Kozlosky, J.J. Peschon, J.L. Slack, M.F. Wolfson, B.J. Castner, K.L. Stocking, P. Reddy, S. Srinivasan, N. Nelson, N. Boiani, K.A. Schooley, M. Gerhart, R. Davis, J.N. Fitzner, R.S. Johnson, R.J. Paxton, C.J. March, and D.P. Cerretti. 1997. A metalloproteinase disintegrin that releases tumour-necrosis factor-alpha from cells. Nature. 385:729-733. doi:10.1038/385729a0.
Blaydon, D.C., P. Biancheri, W.-L. Di, V. Plagnol, R.M. Cabral, M.A. Brooke, D.A. van Heel, F. Ruschendorf, M. Toynbee, A. Walne, E.A. O'Toole, J.E. Martin, K. Lindley, T. Vulliamy, D.J. Abrams, T.T. MacDonald, J.I. Harper, and D.P. Kelsell. 2011. Inflammatory skin and bowel disease linked to ADAM17 deletion. N. Engl. J. Med. 365:1502-1508. doi:10.1056/NEJMoa1100721.
Brüggemann, M., and K. Rajewsky. 1982. Regulation of the antibody response against hapten-coupled erythrocytes by monoclonal antihapten antibodies of various isotypes. Cell. Immunol. 71:365-373. doi:10.1016/0008-8749(82)90270-2.
Budagian, V., E. Bulanova, Z. Orinska, A. Ludwig, S. Rose-John, P. Saftig, E.C. Borden, and S. Bulfone-Paus. 2004. Natural soluble interleukin-15Ralpha is generated by cleavage that involves the tumor necrosis factor-alpha-converting enzyme (TACE/ADAM17). J. Biol. Chem. 279:40368-40375. doi:10.1074/jbc.M404125200.
Cerottini, J.C., P.J. McConahey, and F.J. Dixon. 1969. The immunosuppressive effect of passively administered antibody IgG fragments. The Journal of Immunology. 102:1008-1015.
Charbonneau, M., K. Harper, F. Grondin, M. Pelmus, P.P. McDonald, and C.M. Dubois. 2007. Hypoxia-inducible factor mediates hypoxic and tumor necrosis factor alpha-induced increases in tumor necrosis factor-alpha converting enzyme/ADAM17 expression by synovial cells. J. Biol. Chem. 282:33714-33724. doi:10.1074/jbc.M704041200.
Chen, X., R. Li, Z. Pan, C. Qian, Y. Yang, R. You, J. Zhao, P. Liu, L. Gao, Z. Li, Q. Huang, L. Xu, J. Tang, Q. Tian, W. Yao, L. Hu, X. Yan, X. Zhou, Y. Wu, K. Deng, Z. Zhang, Z. Qian, Y. Chen, and L. Ye. 2020. Human monoclonal antibodies block the binding of SARS-CoV-2 spike protein to angiotensin converting enzyme 2 receptor. Cell. Mol. Immunol. 579:265-3. doi:10.1038/s41423-020-0426-7.
Chen YY, Liu D, Zhang P, et al. 2016. Impact of ACE2 gene polymorphism on antihypertensive efficacy of ACE inhibitors. J Hum Hypertens; 30: 766-71 Chen Q, Tang X, Yu CQ, et al. 2010. Correlation of angiotensin-converting enzyme 2 gene polymorphism with antihypertensive effects of benazepril. Beijing Da Xue Xue Bao; 42: 293-298
Daëron, M., and R. Lesourne. 2006. Negative signaling in Fc receptor complexes. Adv. Immunol. 89:39-86. doi:10.1016/S0065-2776(05)89002-9.
Epelman, S., W.H.W. Tang, S.Y. Chen, F. Van Lente, G.S. Francis, and S. Sen. 2008. Detection of soluble angiotensinconverting enzyme 2 in heart failure: insights into the endogenous counter-regulatory pathway of the reninangiotensin-aldosterone system. J. Am. Coll. Cardiol. 52:750-754. doi:10.1016/j.jacc.2008.02.088.
Fan XH, Wang YB, Wang H, et al. Polymorphisms of angiotensin-converting enzyme (ACE) and ACE2 are not associated with orthostatic blood pressure dysregulation in hypertensive patients. Acta Pharmacol Sin 2009; 30: 1237-44
Feldmann, M., R.N. Maini, J.N. Woody, S.T. Holgate, G. Winter, M. Rowland, D. Richards, and T. Hussell. 2020. Trials of antitumour necrosis factor therapy for COVID-19 are urgently needed. Lancet. doi:10.1016/S0140-6736(20)30858-8.
Garbers, C., N. Jänner, A. Chalaris, M.L. Moss, D.M. Floss, D. Meyer, F. Koch-Nolte, S. Rose-John, and J. Scheller. 2011. Species specificity of ADAM10 and ADAM17 proteins in interleukin-6 (IL-6) trans-signaling and novel role of ADAM10 in inducible IL-6 receptor shedding. J. Biol. Chem. 286:14804-14811. doi:10.1074/jbc.M111.229393.
Getahun, A., and B. Heyman. 2009. Studies on the mechanism by which antigen-specific IgG suppresses primary antibody responses: evidence for epitope masking and decreased localization of antigen in the spleen. Scand. J. Immunol. 70:277-287. doi:10.1111/j.1365-3083.2009.02298.x.
Glowacka, I., S. Bertram, P. Herzog, S. Pfefferle, I. Steffen, M.O. Muench, G. Simmons, H. Hofmann, T. Kuri, F. Weber, J. Eichler, C. Drosten, and S. Pöhlmann. 2010. Differential downregulation of ACE2 by the spike proteins of severe acute respiratory syndrome coronavirus and human coronavirus NL63. Journal of Virology. 84:1198-1205. doi:10.1128/JVI.01248-09.
Haga, S., N. Nagata, T. Okamura, N. Yamamoto, T. Sata, N. Yamamoto, T. Sasazuki, and Y. Ishizaka. 2010. TACE antagonists blocking ACE2 shedding caused by the spike protein of SARS-CoV are candidate antiviral compounds. Antiviral Res. 85:551-555. doi:10.1016/j.antiviral.2009.12.001.
Haga, S., N. Yamamoto, C. Nakai-Murakami, Y. Osawa, K. Tokunaga, T. Sata, N. Yamamoto, T. Sasazuki, and Y. Ishizaka. 2008. Modulation of TNF-alpha-converting enzyme by the spike protein of SARS-CoV and ACE2 induces TNF-alpha production and facilitates viral entry. Proc. Natl. Acad. Sci. U.S.A. 105:7809-7814. doi:10.1073/pnas.0711241105.
Henry, C., and N.K. Jerne. 1968. Competition of 19S and 7S antigen receptors in the regulation of the primary immune response. J Exp Med. 128:133-152. doi:10.1084/jem.128.1.133.
Henry, C., M. Zaizafoun, E. Stock, S. Ghamande, A.C. Arroliga, and H.D. White. 2018. Impact of angiotensin-converting enzyme inhibitors and statins on viral pneumonia. Proc (Bayl Univ Med Cent). 31:419-423. doi:10.1080/08998280.2018.1499293.
Heurich, A., H. Hofmann-Winkler, S. Gierer, T. Liepold, O. Jahn, and S. Pöhlmann. 2014. TMPRSS2 and ADAM17 cleave ACE2 differentially and only proteolysis by TMPRSS2 augments entry driven by the severe acute respiratory syndrome coronavirus spike protein. Journal of Virology. 88:1293-1307. doi:10.1128/JVI.02202-13.
Heyman, B. 1999. Antibody feedback suppression: towards a unifying concept? Immunol. Lett. 68:41-45. doi:10.1016/s0165-2478(99)00028-0.
Heyman, B., and H. Wigzell. 1984. Immunoregulation by monoclonal sheep erythrocyte-specific IgG antibodies: suppression is correlated to level of antigen binding and not to isotype. The Journal of Immunology. 132:1136-1143.
Heyman, B., and H. Wigzell. 1985. Specific IgM enhances and IgG inhibits the induction of immunological memory in mice. Scand. J. Immunol. 21:255-266. doi:10.1111/j.1365-3083.1985.tb01428.x.
Hilliard, L.M., A.K. Sampson, R.D. Brown, and K.M. Denton. 2013. The "his and hers" of the renin-angiotensin system. Curr. Hypertens. Rep. 15:71-79. doi:10.1007/s11906-012-0319-y.
Hisatake, S., S. Kiuchi, T. Kabuki, T. Oka, S. Dobashi, and T. Ikeda. 2017. Serum angiotensin-converting enzyme 2 concentration and angiotensin-(1-7) concentration in patients with acute heart failure patients requiring emergency hospitalization. Heart Vessels. 32:303-308. doi:10.1007/s00380-016-0877-z.
Hoffmann, M., H. Kleine-Weber, S. Schroeder, N. Krüger, T. Herrler, S. Erichsen, T.S. Schiergens, G. Herrler, N.-H. Wu, A. Nitsche, M.A. Muller, C. Drosten, and S. Pöhlmann. 2020. SARS-CoV-2 Cell Entry Depends on ACE2 and TMPRSS2 and Is Blocked by a Clinically Proven Protease Inhibitor. Cell. 181:271-280.e8. doi:10.1016/j.cell.2020.02.052.
Hofmann, H., K. Pyrc, L. van der Hoek, M. Geier, B. Berkhout, and S. Pöhlmann. 2005. Human coronavirus NL63 employs the severe acute respiratory syndrome coronavirus receptor for cellular entry. Proc Natl Acad Sci USA. 102:7988- 7993. doi:10.1073/pnas.0409465102.
Huang, C., Y. Wang, X. Li, L. Ren, J. Zhao, Y. Hu, L. Zhang, G. Fan, J. Xu, X. Gu, Z. Cheng, T. Yu, J. Xia, Y. Wei, W. Wu, X. Xie, W. Yin, H. Li, M. Liu, Y. Xiao, H. Gao, L. Guo, J. Xie, G. Wang, R. Jiang, Z. Gao, Q. Jin, J. Wang, and B. Cao. 2020. Clinical features of patients infected with 2019 novel coronavirus in Wuhan, China. Lancet. 395:497-506. doi:10.1016/S0140-6736(20)30183-5.
Hussell, T., A. Pennycook, and P.J. Openshaw. 2001. Inhibition of tumor necrosis factor reduces the severity of virusspecific lung immunopathology. Eur. J. Immunol. 31:2566-2573. doi:10.1002/1521-4141(200109)31:9<2566::ai-dimmu2566> 3.0.co;2-I.
Jia, H.P., D.C. Look, P. Tan, L. Shi, M. Hickey, L. Gakhar, M.C. Chappell, C. Wohlford-Lenane, and P.B. McCray. 2009. Ectodomain shedding of angiotensin converting enzyme 2 in human airway epithelia. Am. J. Physiol. Lung Cell Mol. Physiol. 297:L84-96. doi:10.1152/ajplung.00071.2009.
Karlsson, M.C., A. Getahun, and B. Heyman. 2001a. FcgammaRIIB in IgG-mediated suppression of antibody responses: different impact in vivo and in vitro. The Journal of Immunology. 167:5558-5564. doi:10.4049/jim-munol.167.10.5558.
Karlsson, M.C., S. Wernersson, T. Diaz de Stahl, S. Gustavsson, and B. Heyman. 1999. Efficient IgG-mediated suppression of primary antibody responses in Fcgamma receptor-deficient mice. Proc Natl Acad Sci USA. 96:2244-2249. doi:10.1073/pnas.96.5.2244.
Karlsson, M.C., T. Diaz de Stahl, and B. Heyman. 2001b. IgE-mediated suppression of primary antibody responses in vivo. Scand. J. Immunol. 53:381-385. doi:10.1046/j.1365-3083.2001.00886.x.
Khan, A., C. Benthin, B. Zeno, T.E. Albertson, J. Boyd, J.D. Christie, R. Hall, G. Poirier, J.J. Ronco, M. Tidswell, K. Hardes, W.M. Powley, T.J. Wright, S.K. Sie-derer, D.A. Fairman, D.A. Lipson, A.I. Bayliffe, and A.L. Lazaar. 2017. A pilot clinical trial of recombinant human angiotensin-converting enzyme 2 in acute respiratory distress syndrome. Crit Care. 21:234-9. doi:10.1186/s13054-017-1823-x.
Kissler, S.M., C. Tedijanto, E. Goldstein, Y.H. Grad, and M. Lipsitch. 2020. Projecting the transmission dynamics of SARSCoV- 2 through the postpandemic period. Science. eabb5793. doi:10.1126/science.abb5793.
Kruse, R.L. 2020. Therapeutic strategies in an outbreak scenario to treat the novel coronavirus originating in Wuhan, China. F1000Res. 9:72. doi:10.12688/f1000research.22211.2.
Lan, J., J. Ge, J. Yu, S. Shan, H. Zhou, S. Fan, Q. Zhang, X. Shi, Q. Wang, L. Zhang, and X. Wang. 2020. Structure of the SARSCoV- 2 spike receptor-binding domain bound to the ACE2 receptor. Nature. 579:270-6. doi:10.1038/s41586-020- 2180-5.
Lew, R.A., F.J. Warner, I. Hanchapola, M.A. Yarski, J. Ramchand, J. Manohar, L.M. Burrell, and A.I. Smith. 2008. Angiotensinconverting enzyme 2 catalytic activity in human plasma is masked by an endogenous inhibitor. Exp. Physiol. 93:685- 693. doi:10.1113/expphysiol.2007.040352.
Li, W., C. Zhang, J. Sui, J.H. Kuhn, M.J. Moore, S. Luo, S.-K. Wong, I.-C. Huang, K. Xu, N. Vasilieva, A. Murakami, Y. He, W.A. Marasco, Y. Guan, H. Choe, and M. Farzan. 2005. Receptor and viral determinants of SARS-coronavirus adaptation to human ACE2. EMBO J. 24:1634-1643. doi:10.1038/sj.emboj.7600640.
Li K., Wu M., Huang B., Zhong A., Li L., Cai Y., Wu L., Zhu M., Li J., Wang Z., Wu W., Li W., Bosco B., Gan Z., Wang Z., Qia Q, Wu J., Wang Q., Wang S., Xia X. 2020. The Dynamic Changes of Antibodies against SARS-CoV-2 during the Infection and Recovery of COVID-19. medRxiv preprint doi: https://doi.org/10.1101/2020.05.18.20105155
Liang, Y., H. Deng, S. Bi, Z. Cui, L. A, D. Zheng, and Y. Wang. 2015. Urinary angiotensin converting enzyme 2 increases in patients with type 2 diabetic mellitus. Kidney Blood Press. Res. 40:101-110. doi:10.1159/000368486.
Liu, J., H. Ji, W. Zheng, X. Wu, J.J. Zhu, A.P. Arnold, and K. Sandberg. 2010. Sex differences in renal angiotensin converting enzyme 2 (ACE2) activity are 17β-oestradiol-dependent and sex chromosome-independent. Biol Sex Differ. 1:6-11. doi:10.1186/2042-6410-1-6.
Liu, W., L. Liu, G. Kou, Y. Zheng, Y. Ding, W. Ni, Q. Wang, L. Tan, W. Wu, S. Tang, Z. Xiong, and S. Zheng. 2020. Evaluation of Nucleocapsid and Spike Protein-based ELISAs for detecting antibodies against SARS-CoV-2. J. Clin. Micro-biol. doi:10.1128/JCM.00461-20.
Luo Y, Liu C, Guan T, Li Y, Lai Y, Li F, Zhao H, Maimaiti T, Zeyaweiding A. 2019.Association of ACE2 genetic polymorphisms with hypertension-related target organ damages in south Xinjiang Hypertens Res; 42(5): 681-689.
Malard L, Kakinami L, O'Loughlin J, Roy-Gagnon MH, Labbe A, Pilote L, Hamet P, Tremblay J, Paradis G. 2013.The association between the angiotensin-converting enzyme-2 gene and blood pressure in a cohort study of adolescents. BMC Med Genet; 14: 117.
Meyer, B., C. Drosten, and M.A. Muller. 2014. Serological assays for emerging coronaviruses: challenges and pitfalls. Virus Res. 194:175-183. doi:10.1016/j.vi-rusres.2014.03.018.
Meyer-Hermann, M. 2019. Injection of Antibodies against Immunodominant Epitopes Tunes Germinal Centers to Generate Broadly Neutralizing Antibodies. CellReports. 29:1066-1073.e5. doi:10.1016/j.celrep.2019.09.058.
Moss, M.L., and D. Minond. 2017. Recent Advances in ADAM17 Research: A Promising Target for Cancer and Inflammation. Mediators Inflamm. 2017:9673537-21. doi:10.1155/2017/9673537.
Moss, M.L., L. Sklair-Tavron, and R. Nudelman. 2008. Drug insight: tumor necrosis factor-converting enzyme as a pharmaceutical target for rheumatoid arthritis. Nat Clin Pract Rheumatol. 4:300-309. doi:10.1038/ncprheum0797.
Moss, M.L., S.L. Jin, M.E. Milla, D.M. Bickett, W. Burkhart, H.L. Carter, W.J. Chen, W.C. Clay, J.R. Didsbury, D. Hassler, C.R. Hoffman, T.A. Kost, M.H. Lambert, M.A. Leesnitzer, P. McCauley, G. McGeehan, J. Mitchell, M. Moyer, G. Pa-hel, W. Rocque, L.K. Overton, F. Schoenen, T. Seaton, J.L. Su, and J.D. Becherer. 1997. Cloning of a disintegrin metalloproteinase that processes precursor tumour-necrosis factor-alpha. Nature. 385:733-736. doi:10.1038/385733a0.
Moller, G. 1985. Antibody-mediated suppression of the immune response is determinant specific. Eur. J. Immunol. 15:409- 412. doi:10.1002/eji.1830150420.
Newton R. C. , E. C. Bradley , R. S. Levy, D. Doval, S. Bondarde , T. P. SahooD. Lokanatha , P. K. Julka , R. Nagarkar , S. M. Friedman. 2010. Clinical benefit of INCB7839, a potent and selective ADAM inhibitor, in combination with trastuzumab in patients with metastatic HER2+ breast cancer. Journal of Clinical Oncology. vol. 28, 15_Supplement, pp. 3025-3025,.
Nicholas, R., and S.C. Sinclair. 1969. Regulation of the immune response. I. Reduction in ability of specific antibody to inhibit long-lasting IgG immunological priming after removal of the Fc fragment. J Exp Med. 129:1183-1201. doi:10.1084/jem.129.6.1183.
Nimmerjahn, F., and J.V. Ravetch. 2010. Antibody-mediated modulation of immune responses. Immunol. Rev. 236:265- 275. doi:10.1111/j.1600-065X.2010.00910.x.
Niu W, Qi Y, Hou S, Zhou W, Qiu C. 2007. Correlation of angiotensin-converting enzyme 2 gene polymorphisms with stage 2 hypertension in Han Chinese. Transl Res; 150: 374-80.
Patel, I.R., M.G. Attur, R.N. Patel, S.A. Stuchin, R.A. Abagyan, S.B. Abramson, and A.R. Amin. 1998. TNF-alpha convertase enzyme from human arthritis-affected cartilage: isolation of cDNA by differential display, expression of the active enzyme, and regulation of TNF-alpha. The Journal of Immunology. 160:4570-4579.
Patnaik M, Pati P, Swain SN, et al. 2014. Association of angiotensin-752 converting enzyme and angiotensin-converting enzyme-2 gene polymorphisms with essential hypertension in the population of Odisha, India. Ann Hum Biol; 41: 143-150
Pedersen, K.B., H. Chodavarapu, C. Porretta, L.K. Robinson, and E. Laz-artigues. 2015. Dynamics of ADAM17-Mediated Shedding of ACE2 Applied to Pancreatic Islets of Male db/db Mice. Endocrinology. 156:4411-4425. doi:10.1210/en.2015-1556.
Pinheiro DS, Santos RS, Veiga Jardim PCB, Silva EG, Reis AAS, Pedrino GR, Ulhoa CJ. 2019. The combination of ACE I/D and ACE2 G8790A polymorphisms revels susceptibility to hypertension: A genetic association study in Brazilian patients PLoS One; 14(8):759 e0221248.
Poglitsch, M., O. Domenig, C. Schwager, S. Stranner, B. Peball, E. Janzek, B. Wagner, H. Jungwirth, H. Loibner, and M. Schuster. 2012. Recombinant Expression and Characterization of Human and Murine ACE2: Species-Specific Activation of the Alternative Renin-Angiotensin-System. Int J Hypertens. 2012:428950-8. doi:10.1155/2012/428950.
Ramchand, J., S.K. Patel, P.M. Srivastava, O. Farouque, and L.M. Burrell. 2018. Elevated plasma angiotensin converting enzyme 2 activity is an independent predictor of major adverse cardiac events in patients with obstructive coronary artery disease. PLoS ONE. 13:e0198144. doi:10.1371/journal.pone.0198144.
Reth, M., G. Kelsoe, and K. Rajewsky. 1981. Idiotypic regulation by isologous monoclonal anti-idiotope antibodies. Nature. 290:257-259. doi:10.1038/290257a0.
Rice, G.I., A.L. Jones, P.J. Grant, A.M. Carter, A.J. Turner, and N.M. Hooper. 2006. Circulating activities of angiotensinconverting enzyme, its homolog, angiotensin-converting enzyme 2, and neprilysin in a family study. Hypertension. 48:914-920. doi:10.1161/01.HYP.0000244543.91937.79.
Richards, F.M., C.J. Tape, D.I. Jodrell, and G. Murphy. 2012. Anti-tumour effects of a specific anti-ADAM17 antibody in an ovarian cancer model in vivo. PLoS ONE. 7:e40597. doi:10.1371/journal.pone.0040597.
Rios-Doria, J., D. Sabol, J. Chesebrough, D. Stewart, L. Xu, R. Tammali, L. Cheng, Q. Du, K. Schifferli, R. Rothstein, C.C. Leow, J. Heidbrink-Thompson, X. Jin, C. Gao, J. Friedman, B. Wilkinson, M. Damschroder, A.J. Pierce, R.E. Hol-lingsworth, D.A. Tice, and E.F. Michelotti. 2015. A Monoclonal Antibody to ADAM17 Inhibits Tumor Growth by Inhibiting EGFR and Non-EGFR-Mediated Pathways. Mol Cancer Ther. 14:1637-1649. doi:10.1158/1535-7163.MCT-14-1040.
Robbian D.F., Gaebler C. Muecksc F., Lorenzi J. C. C. , Wang Z., Cho A., Agudelo M., Barnes C. O., Finkin S., Hagglof T., Oliveira T.Y., Viant C., Hurley A.,Millard K. G., Kost R. G., Cipolla M., Gazumyan A., Gordon K., Bianchini F., Chen S. T., Ramos V., Patel R., Dizon J., Shimeliovich I., Mendoza P., Hartweger H., Nogueira L. , Pack M., Horowitz J., Schmidt F., Weisblum Y., Hoffmann H-H., Michailidis E., Ashbrook A. W., Waltari E., Pak J. E., Huey-Tubma K. E. , Koranda N., Hoffman P. R., West A. P., Rice C. M., Hatziioannou T., Bjorkman P. J., BieniaszP. D. Caskey M., Nussenzweig M. C.; 2020. Convergent Antibody Responses to SARS-CoV-2 Infection in Convalescent Individuals. bioRxiv preprint doi: https://doi.org/10.1101/2020.05.13.092619
Safford, J.W., and S. Tokuda. 1971. Antibody-mediated suppression of the immune response: effect on the development of immunologic memory. The Journal of Immunology. 107:1213-1225.
Somineni, H.K., G.P. Boivin, and K.M. Elased. 2014. Daily exercise training protects against albuminuria and angiotensin converting enzyme 2 shedding in db/db diabetic mice. J. Endocrinol. 221:235-251. doi:10.1530/JOE-13-0532.
Song, W., M. Gui, X. Wang, and Y. Xiang. 2018. Cryo-EM structure of the SARS coronavirus spike glycoprotein in complex with its host cell receptor ACE2. PLoS Pathog. 14:e1007236. doi:10.1371/journal.ppat.1007236.
Tai, W., L. He, X. Zhang, J. Pu, D. Voronin, S. Jiang, Y. Zhou, and L. Du. 2020. Characterization of the receptor-binding domain (RBD) of 2019 novel coronavirus: implication for development of RBD protein as a viral attachment inhibitor and vaccine. Cell. Mol. Immunol. 7:226-8. doi:10.1038/s41423-020-0400-4.
Takayanagi, T., S.J. Forrester, T. Kawai, T. Obama, T. Tsuji, K.J. Elliott, E. Nuti, A. Rossello, H.F. Kwok, R. Scalia, V. Rizzo, and S. Eguchi. 2016. Vascular ADAM17 as a Novel Therapeutic Target in Mediating Cardiovascular Hypertrophy and Perivascular Fibrosis Induced by Angiotensin II. Hypertension. 68:949-955. doi:10.1161/HYPERTENSIONAHA.116.07620.
Tao, T.W., and J.W. Uhr. 1966. Capacity of pepsin-digested antibody to inhibit antibody formation. Nature. 212:208-209. doi:10.1038/212208a0.
Úri, K., M. Fagyas, A. Kertész, A. Borbély, C. Jenei, O. Bene, Z. Csanádi, W.J. Paulus, I. Édes, Z. Papp, A. Tóth, and E. Lizanecz. 2016. Circulating ACE2 activity correlates with cardiovascular disease development. J Renin Angiotensin Aldosterone Syst. 17. doi:10.1177/1470320316668435.
Úri, K., M. Fagyas, I. Mányiné Siket, A. Kertész, Z. Csanádi, G. Sándorfi, M. Clemens, R. Fedor, Z. Papp, I. Édes, A. Tóth, and E. Lizanecz. 2014. New perspectives in the renin-angiotensin-aldosterone system (RAAS) IV: circulating ACE2 as a biomarker of systolic dysfunction in human hypertension and heart failure. PLoS ONE. 9:e87845. doi:10.1371/journal.pone.0087845.
Walls, A.C., M.A. Tortorici, B. Frenz, J. Snijder, W. Li, F.A. Rey, F. DiMaio, B.-J. Bosch, and D. Veesler. 2016. Glycan shield and epitope masking of a coronavirus spike protein observed by cryo-electron microscopy. Nature Structural & Molecular Biology. 23:899-905. doi:10.1038/nsmb.3293.
Walls, A.C., M.A. Tortorici, J. Snijder, X. Xiong, B.-J. Bosch, F.A. Rey, and D. Veesler. 2017. Tectonic conformational changes of a coronavirus spike glycoprotein promote membrane fusion. Proc. Natl. Acad. Sci. U.S.A. 114:11157-11162. doi:10.1073/pnas.1708727114.
Walls, A.C., X. Xiong, Y.-J. Park, M.A. Tortorici, J. Snijder, J. Quispe, E. Camer-oni, R. Gopal, M. Dai, A. Lanzavecchia, M. Zambon, F.A. Rey, D. Corti, and D. Veesler. 2019. Unexpected Receptor Functional Mimicry Elucidates Activation of Coronavirus Fusion. Cell. 176:1026-1039.e15. doi:10.1016/j.cell.2018.12.028.
Walls, A.C., Y.-J. Park, M.A. Tortorici, A. Wall, A.T. McGuire, and D. Veesler. 2020. Structure, Function, and Antigenicity of the SARS-CoV-2 Spike Glycoprotein. Cell. 181:281-292.e6. doi:10.1016/j.cell.2020.02.058.
Wang, L., W. He, X. Yu, D. Hu, M. Bao, H. Liu, J. Zhou, and H. Jiang. 2020. Coronavirus disease 2019 in elderly patients: Characteristics and prognostic factors based on 4-week follow-up. J. Infect. doi:10.1016/j.jinf.2020.03.019.
Wang, M., W. Zhang, Y. Zhou, and X. Zhou. 2014. Association between serum angiotensin-converting enzyme 2 levels and postoperative myocardial infarction following coronary artery bypass grafting. Exp Ther Med. 7:1721-1727. doi:10.3892/etm.2014.1640.
Witters, L., P. Scherle, S. Friedman, J. Fridman, E. Caulder, R. Newton, and A. Lipton. 2008. Synergistic inhibition with a dual epidermal growth factor receptor/HER-2/neu tyrosine kinase inhibitor and a disintegrin and metalloprotease inhibitor. Cancer Res. 68:7083-7089. doi:10.1158/0008-5472.CAN-08-0739.
Wong, E., T. Cohen, E. Romi, M. Levin, Y. Peleg, U. Arad, A. Yaron, M.E. Milla, and I. Sagi. 2016. Harnessing the natural inhibitory domain to control TNFα Converting Enzyme (TACE) activity in vivo. Scientific Reports. 6:35598-12. doi:10.1038/srep35598.
Wrapp, D., N. Wang, K.S. Corbett, J.A. Goldsmith, C.-L. Hsieh, O. Abiona, B.S. Graham, and J.S. McLellan. 2020. Cryo-EM structure of the 2019-nCoV spike in the prefusion conformation. Science. 367:1260-1263. doi:10.1126/science.abb2507.
Wu.F., Aojie Wang, Mei Liu, Qimin Wang, Jun Chen, Shuai Xia, Yun Ling, Yuling Zhang, Jingna Xun, Lu Lu, Shibo Jiang, Hongz hou Lu, Yumei Wen, Jinghe Huang. 2020. Neutralizing antibody responses to SARS-CoV-2 in a COVID-19 recovered patient cohort and their implications. medRxiv. Preprint. doi: https://doi.org/10.1101/2020.03.30.20047365
Wu, K., W. Li, G. Peng, and F. Li. 2009. Crystal structure of NL63 respiratory coronavirus receptor-binding domain complexed with its human receptor. Proc. Natl. Acad. Sci. U.S.A. 106:19970-19974. doi:10.1073/pnas.0908837106.
Xiao, F., S. Hiremath, G. Knoll, J. Zimpelmann, K. Srivaratharajah, D. Jadhav, D. Fergusson, C.R.J. Kennedy, and K.D. Burns. 2012. Increased urinary angiotensin-converting enzyme 2 in renal transplant patients with diabetes. PLoS ONE. 7:e37649. doi:10.1371/journal.pone.0037649.
Yang, P., H. Gu, Z. Zhao, W. Wang, B. Cao, C. Lai, X. Yang, L. Zhang, Y. Duan, S. Zhang, W. Chen, W. Zhen, M. Cai, J.M. Penninger, C. Jiang, and X. Wang. 2014. Angiotensin-converting enzyme 2 (ACE2) mediates influenza H7N9 vi-rusinduced acute lung injury. Scientific Reports. 4:7027-6. doi:10.1038/srep07027.
Yi L, Gu YH, Wang XL, et al. 2006. Association of ACE, ACE2 and UTS2 polymorphisms with essential hypertension in Han and Dongxiang populations from North-Western China. J. Int Med Res; 34: 272-83.
Yu, L., K. Yuan, H.T.A. Phuong, B.M. Park, and S.H. Kim. 2016. Angiotensin-(1-5), an active mediator of renin-angiotensin system, stimulates ANP secretion via Mas receptor. Peptides. 86:33-41. doi:10.1016/j.peptides.2016.09.009.
Zarnitsyna, V.I., A.H. Ellebedy, C. Davis, J. Jacob, R. Ahmed, and R. Antia. 2015. Masking of antigenic epitopes by antibodies shapes the humoral immune response to influenza. Philos. Trans. R. Soc. Lond., B, Biol. Sci. 370:20140248. doi:10.1098/rstb.2014.0248.
Zhang, Y., M. Meyer-Hermann, L.A. George, M.T. Figge, M. Khan, M. Goodall, S.P. Young, A. Reynolds, F. Falciani, A. Waisman, C.A. Notley, M.R. Ehrenstein, M. Kosco-Vilbois, and K.-M. Toellner. 2013. Germinal center B cells govern their own fate via antibody feedback. J Exp Med. 210:457-464. doi:10.1084/jem.20120150.
Zhao, J., Q. Yuan, H. Wang, W. Liu, X. Liao, Y. Su, X. Wang, J. Yuan, T. Li, J. Li, S. Qian, C. Hong, F. Wang, Y. Liu, Z. Wang, Q. He, Z. Li, B. He, T. Zhang, Y. Fu, S. Ge, L. Liu, J. Zhang, N. Xia, and Z. Zhang. 2020. Antibody responses to SARS-CoV-2 in patients of novel coronavirus disease 2019. Clin. Infect. Dis. doi:10.1093/cid/

## Claims

1. ADAM17 inhibitor for use in the prevention and/or treatment of COVID-19 in a subject.

2. The ADAM17 inhibitor for use according to claim 1, wherein the subject shows at least low abundance of SARS-CoV-2 neutralizing antibodies upon vaccination or natural infection, more preferably SARS-CoV-2 spike protein neutralizing antibodies, more preferably SARS-CoV-2 RBD protein specific antibodies, more preferably SARS-CoV-2 RBD Ace2 binding site specific IgG, IgA and/or IgM antibodies.

3. The ADAM17 inhibitor for use according to any one of the preceding claims wherein a SARS-CoV-2 infected subject is asymptomatic, or has developed mild disease at an early stage of disease onset.

4. The ADAM17 inhibitor for use according to any one of the preceding claims wherein a SARS-CoV-2 infected subject has developed inflammation symptoms.

5. The ADAM17 inhibitor for use according to any one of the preceding claims wherein a SARS-CoV-2 infected subject shows an increased amount of TNF-alpha in serum or plasma of at least 1.5 fold and/or of Angiotensin II in serum or plasma of at least 2 fold.

6. The ADAM17 inhibitor for use according to any one of the preceding claims wherein the subject is of risk to develop severe COVID-19 disease outcome.

7. The ADAM17 inhibitor for use according to any one of the preceding claims wherein the subject shows an increased amount of soluble Ace2 in human serum or plasma of at least 1.5 fold and/or shows a concentration of soluble Ace2 in human serum or plasma of at least 1 ng/ ml.

8. The ADAM17 inhibitor for use according to any one of the preceding claims wherein the subject has a genetic predisposition for high Ace2 receptor expression or Ace2 receptor shedding.

9. The ADAM17 inhibitor for use according to any one of the preceding claims, wherein the subject suffers from a condition or disease selected from cardiovascular disease, diabetes, obstructive coronary artery disease, systolic dysfunction in human hypertension and heart failure, high age of at least 60 years, male, waste to hip ratio of at least 0.87 cm, obesity with a BMI of at least 34.7 kg/m², a systolic blood pressure of at least 134.7 mm Hg, a faster glucose level of at least 5.24 mmol/L, a concentration of cholesterol of at least 5.26 mmol/L, a concentration of triglycerides of at least 1.54 mmol/L.

10. The ADAM17 inhibitor for use according to any one of the preceding claims, wherein the inhibitor is a chemical compound, preferably a chemical compound selected from methyl (6S,7S)-7-(hydroxycarbamoyl)-6-(4-phenylpiperazine-1-carbonyl)-5-azaspiro[2.5]octane-5-carboxylate; methyl (5S,6S)-5-(hydroxycarbamoyl)-6-(4-phenyl3,6-dihydro-2H-pyridine-1-carbonyl)-7-azaspiro[2.5]octane-7-carboxylate; (3S)-N-Hydroxy-4-((4-((4-hydroxybut-2-ynyl)oxy)phenyl)sulfonyl)-2,2-dimethylthiomorpholine-3-carboxamide(3S)-N-Hydroxy-4-((4-((4-hydroxybut-2-ynyl)oxy)phenyl)sulfonyl)-2,2-dimethylthiomorpholine-3-carboxamide; (3S)-4-[[4-(2-Butyn-1-yloxy)phenyl]sulfonyl]-N-hydroxy-2,2-dimethyl-3-thiomorpholinecarbox-amide, Acetamide, 2-(2,2-dimethyl-5-oxo-1,3-dioxolan-4-ylidene)-N-((4-fluorophenyl)methyl)-N-methoxy-.

11. The ADAM17 inhibitor for use according to any one of the preceding claims, wherein the inhibitor is used in combination with a vaccine, preferably an active immunization vaccine.

12. The ADAM17 inhibitor for use according to claim 11, wherein the ADAM17 inhibitor is administered one to seven days prior to vaccination.

13. A pharmaceutical composition comprising an ADAM17 inhibitor as an active ingredient for use in the prevention and/or treatment of COVID-19 in a subject.

14. A kit of parts for use in the prevention and/or treatment of COVID-19 in a subject comprising i) an ADAM17 inhibitor and ii) a vaccine.

15. A pharmaceutical composition comprising an ADAM17 inhibitor and vaccine for active immunization.

16. A kit of parts comprising i) an ADAM17 inhibitor and ii) a vaccine for active immunization.
